# EUROPEAN PATENT APPLICATION

(11) **EP 4 085 909 A1**
(43) Date of publication of application: **09.11.2022**
(21) Application number: 21382409.7
(22) Date of filing: 06.05.2021
(51) Int. Cl.: A61K 31/381, A61P 25/28

(54) **METHODS AND COMPOSITIONS FOR THE TREATMENT OF DISORDERS CHARACTERIZED BY A KIDINS220 DYSFUNCTION IN A SUBJECT**

(71) Applicant: Consejo Superior de Investigaciones Cientificas, 28006 Madrid (ES); Universidad Autónoma de Madrid, 28049 Madrid (ES); Consorcio Centro de Investigación Biomédica en Red del Área de Enfermedades Neurodegenerativas (CIBERNED), 28031 Madrid (ES)
(72) Inventor: IGLESIAS VACAS, Teresa, 28049 Madrid (ES); CAMPANERO GARCÍA, Miguel R, 28006 Madrid (ES); POSE UTRILLA, Julia, 28049 Madrid (ES); SIMÓN GARCÍA, Ana, 28049 Madrid (ES); LÓPEZ MENÉNDEZ, Celia, 28049 Madrid (ES); SÁNCHEZ-MIRANDA PAJUELO, Luis, 28049 Madrid (ES); DEL PUERTO DEL PINO, Ana María, 28031 Madrid (ES)
(74) Representative: Pons

(57) **Abstract**

The present invention relates to retromer complex activators and compositions for use in the treatment and/or prevention of neurological and psychiatric disorders characterized by Kidins220 dysfunction in a subject. These disorders may be further characterized by ventriculomegaly and/or excitotoxicity. It also relates to methods for screening a retromer complex activator for use in the treatment and/or prevention of said disorders in a subject.

## Description

### TECHNICAL FIELD

The present invention relates to retromer complex activators and compositions for use in the treatment and/or prevention of neurological or psychiatric disorders characterized by Kinase D-interacting substrate of 220 kDa (Kidins220) dysfunction in a subject. It also relates to methods for screening a retromer complex activator for use in the diagnosis, treatment and/or prevention of said disorders in a subject.

### BACKGROUND

Several neurological and psychiatric disorders are characterized by a dysfunction of Kidins220. Most of these disorders are further characterized by increased brain ventricles volume, being hydrocephalus (HCP) the disease with the major manifestation of ventriculomegaly caused by the accumulation of high amounts of cerebrospinal fluid (CSF). The molecules and pathomechanisms underlying cerebral ventricular enlargement are widely unknown.

Additionally, these disorders may also be characterized by excitotoxicity, a process of neuronal death arising from prolonged exposure to glutamate and the associated excessive influx of ions into the neuron that shuts off survival pathways and triggers pro-death cascades. The mechanisms of excitotoxicity and their impact on ventriculomegaly brain damage are also poorly characterized at a molecular level.

Thus, there is a strong need to identify the molecular signatures involved in the development of the disorders characterized by Kidins220 dysfunction and the mechanisms by which said molecular signatures exert their biological role in order to design novel therapies.

### DETAILED DESCRIPTION OF THE INVENTION

Kidins220, also known as ankyrin repeat-rich membrane spanning (ARMS), is a transmembrane protein with a central role in the coordination of receptor crosstalk and the integration of signaling pathways essential for neuronal differentiation, survival and function. Accordingly, the expression of Kidins220 is altered in neurological and psychiatric disorders, including cerebral ischemia, Alzheimer's disease (AD) and Huntington's disease (HD).

Frequent pathogenic processes in these diseases characterized by Kidins220 dysfunction are ventriculomegaly and/or excitotoxicity.

Ventriculomegaly is a non-specific term describing dilatation of the cerebral ventricles. It is a co-morbidity factor in several cerebrovascular, neurological and psychiatric disorders, including HCP, idiopathic normal pressure hydrocephalus (iNPH), ischemic stroke (IS), schizophrenia, Parkinson's disease (PD) and AD.

Excitotoxicity is a critical process in numerous human neuropathologies and particularly in those dementia-associated, such as AD, PD, HD and in those caused by acute brain damage, such as IS and traumatic brain injury (TBI). Excitotoxicity is also observed in certain neuropsychiatric disorders, such as schizophrenia and autism spectrum disorder (ASD).

Kidins220 is an effector of various key neuronal signalling pathways. The *KIDINS220* gene has been recently associated with schizophrenia and with a novel syndrome characterized by spastic paraplegia, intellectual disability, nystagmus and obesity (SINO syndrome), diseases frequently occurring with ventriculomegaly.

However, the specific role of the molecule in these pathologies has not yet been determined.

AQP4 expression in the brain is prominent in the basolateral membranes of ependymocytes lining cerebral ventricles and in the end-feet of astrocytes contacting brain blood vessels and the pia membrane. Aqp4^{-/-} mice show sporadic brain ventricular enlargement, accelerated progression of induced hydrocephalus, and increased basal brain water accumulation, suggesting that AQP4 is critical for controlling whole brain water homeostasis. While studies in iNPH brain revealed loss of astrocytic perivascular AQP4, the underlying mechanisms of AQP4 downregulation have remained unknown. It was also unknown whether AQP4 content at the ependymal ventricular lining is altered in iNPH.

Similarly, evidence supports a role of astrocytic AQP4 in neuroprotection from excitotoxicity *in vitro,* but underlying molecular mechanisms remain poorly explored. Thus, there is an urgent need to identify and characterize the pathogenic mechanisms underlying brain ventricular enlargement and excitotoxicity in different neuropathologies to uncover novel therapeutic strategies.

The inventors unveil herein the critical role of Kidins220 in the regulation of CSF homeostasis. They have discovered that both Kidins220 and the water channel aquaporin-4 (AQP4) are markedly downregulated at the ventricular ependymal lining of iNPH patients, with Kidins220-deficient mice developing ventriculomegaly accompanied by water dyshomeostasis and loss of AQP4 in the brain ventricular ependymal layer and astrocytes.

The inventors have also characterized AQP4 as a novel cargo of the Sorting Nexin 27 (SNX27)-retromer (SNX27-R), a complex that redirects endocytosed plasma membrane proteins (cargos) back to the cell surface, thus avoiding their targeting to lysosomes for degradation. Accordingly, SNX27 silencing decreases AQP4 levels in wild-type astrocytes.

Additionally, the inventors have found that Kidins220 dysfunction promotes a striking and unexpected downregulation of the SNX27-R that results in AQP4 lysosomal degradation, with SNX27 overexpression restoring AQP4 content in Kidins220 deficient astrocytes. Moreover, it is also demonstrated herein that the retromer complex activators increase AQP4 levels in astrocytes.

The inventors have also discovered that excitotoxicity mediated by glutamate N-methyl-D-aspartate (NMDA) receptors (NMDAR) overstimulation in neurons induces retromer destabilization and dysfunction contributing to Kidins220 downregulation, thus confirming the role of the Kidins220-SNX27-R pathway in neuroprotection from excitotoxicity. Furthermore, the experimental results shown herein demonstrate the striking effect of retromer complex activators in maintaining the normal function of the SNX27-R in neurons under NMDA-induced exocitotoxic conditions, hampering Kidins220 and other SNX27-R cargos degradation/downregulation and conferring neuroprotection.

The results obtained confirm the crucial role of these molecules in ventriculomegaly and excitotoxicity, highlighting the SNX27-R pathway, including the Kidins220-SNX27-R and the Kidins220-SNX27-R-AQP4 pathways as a novel therapeutic target for diseases characterized by either or both pathological processes.

In one aspect, the invention discloses a retromer complex activator ("activator of the invention"), or a pharmaceutical composition comprising said activator ("composition of the invention") in a therapeutically effective amount, for use in the treatment of neurological or psychiatric disorders characterized by Kidins220 dysfunction in a subject.

The retromer complex is a protein regulator with a key function in the delivery of proteolytic enzymes to lysosomes. It consists of two subcomplexes that assemble in the cytoplasm; the trimeric cargo recognition core (CRC) complex is composed by vacuolar protein sorting (VPS) 35, 29 and 26; the second subcomplex consists of sorting-nexin (SNX) proteins, either containing phox-homology (PX) or PX and bin/amphiphysin/rvs (BAR) domains. The retromer recycles specific cargos, such as Vps10/Sortilin protein family members or the cation-independent mannose 6-phosphate receptor (CI-MPR). The latter is involved in the delivery of proteolytic enzymes to lysosomes. A point mutation in VPS35 (D620N) is causative of an autosomal dominant form of Parkinson's disease and affects early steps of autophagosome formation. Additionally, VPS35 deficiency alters the distribution of lysosome-associated membrane glycoprotein 2a (Lamp2a) in neurons.

In the present invention, the term "retromer complex activator" or variants thereof refer to any molecule capable of enhancing retromer-mediated trafficking of cargos. The activator of the invention may enhance the trafficking of cargos by mechanisms including, but not limited to, increasing the interaction between components of the retromer complex and/or stabilizing the retromer, the retromer-associated proteins, the interaction between the components of the retromer complex or the interaction between the latter and the retromer-associated proteins.

As used herein, the term "retromer-associated proteins" and any variants thereof refers to any protein that binds to any one of the two retromer subcomplexes and is not a retromer cargo protein. Examples of associated proteins include, without limitation, the WASH complex components FAM21, Strumpelin, SWIP, CCDC53 and WASH1.

The activator of the invention may enhance the retromer-mediated trafficking of cargos by increasing the levels of the biological activity of at least one protein ("protein of interest") selected from the group consisting of: (i) protein components of the retromer; (ii) retromer-associated proteins; and (iii) the retromer's cargo.

The activator of the invention may enhance the retromer-mediated trafficking of cargos by inducing the expression of at least one gene ("gene of interest"). In some embodiments of the invention, the retromer complex activator induces the expression of at least one gene encoding one of the protein components of the retromer. In some embodiments of the invention, the retromer complex activator induces the expression of at least one gene encoding one of the retromer-associated proteins. In some embodiments, the activator of the invention induces the expression of at least one gene encoding one of the retromer cargo components.

The activator of the invention may be a pharmacological retromer complex activator or a biological molecule.

A well-known type of retromer complex activator are pharmacological stabilizers; these stabilizers increase the interaction between the components of the retromer complex, increasing retromer stability and enhancing retromer-mediated trafficking of cargos. They are small molecules, typically with a molecular weight less than about 1000 Daltons, or in some embodiments, less than about 500 Daltons. Examples of these compounds include thiophene thiourea derivatives, as described below.

Biological molecules include large macromolecules and small molecules. By way of non-limiting example, common examples of biomolecules include proteins, carbohydrates, lipids, nucleic acids, primary and secondary metabolites, and natural products. In some embodiments, the activator of the invention is a protein. In some specific embodiments, the activator of the invention is an antibody or fragment thereof. In some embodiments, the activator of the invention is a nucleic acid.

In some embodiments, the activator of the invention is one of the protein components of the retromer. In some embodiments, the activator of the invention is a retromer-associated protein. In some other embodiments, the activator of the invention is one of the protein components of the cargo. In the present invention, the terms polypeptide(s) and protein(s) are used interchangeably.

The present invention relates to the activator or composition of the invention for use in the treatment and/or prevention of neurological or psychiatric disorders characterized by Kidins220 dysfunction in a subject.

The term "neurological disorder" refers to any disease of the central and peripheral nervous system, including the brain, spinal cord, cranial nerves, peripheral nerves, nerve roots, autonomic nervous system, neuromuscular junction and muscles. These disorders include, but are not limited to, epilepsy, AD and other dementias, HCP, iNPH, SINO syndrome, PD, HD, amyotrophic lateral sclerosis, multiple sclerosis, neuroinfections, brain tumours, traumatic disorders of the nervous system due to head trauma, neurological disorders as a result of malnutrition, and cerebrovascular diseases including stroke, migraine and other headache disorders.

As used herein, the term "cerebrovascular disorder" refers to any disorder in which an area of the brain is temporarily or permanently affected by ischemia or bleeding, and one or more of the cerebral blood vessels are involved in the pathological process. Cerebrovascular disorders include, but are not limited to, stroke, migraine, carotid stenosis, vertebral stenosis and intracranial stenosis, aneurysms, and vascular malformations.

The term "psychiatric disorder" or "mental disorder" refers to any medical problem involving (i) significant changes in thinking, emotion, and/or behaviour and (ii) distress and/or problems functioning in social, work, or family activities. There are over two hundred classified types of psychiatric disorders, but some of the most frequently diagnosed ones are bipolar disorder, schizophrenia, ASD, depression, generalized anxiety disorder, etc.

As used herein, the term "Kidins220 dysfunction in a subject" has the same meaning as commonly understood by any person skilled in the art to which this invention belongs, relating to a different function (loss-of-function or gain-of-function) of Kidins220 in a subject when compared to its expected function. By way of non-limiting example, the level of expression of Kidins220 in a biological sample from the subject suffering from a disorder characterized by Kidins220 dysfunction may be decreased with respect to the level of expression of Kidins220 in a biological sample from a subject not suffering from any neurological or psychiatric disorders ("control subject/s"). A detailed explanation of the methods for determining the levels of expression of a protein and the terms used herein is provided in the description of the second and third aspects of the invention.

As used herein, the term "treating" (or "treat" or "treatment") refers to processes involving a slowing, interrupting, arresting, controlling, stopping, reducing, or reversing the progression or severity of an existing symptom, disorder, condition, or disease, but does not necessarily involve a total elimination of all disease-related symptoms, conditions, or disorders. The treatment of a disorder or disease may, for example, lead to a halt in the progression of the disorder or disease (e.g., no deterioration of symptoms) or a delay in the progression of the disorder or disease (in case the halt in progression is of a transient nature only). The "treatment" of a disorder or disease may also lead to a partial response (e.g., amelioration of symptoms) or complete response (e.g., disappearance of symptoms) of the subject/patient suffering from the disorder or disease. Accordingly, the "treatment" of a disorder or disease may also refer to an amelioration of the disorder or disease, which may lead to a halt in the progression of the disorder or disease or a delay in the progression of the disorder or disease. Such a partial or complete response may be followed by a relapse. It is to be understood that a subject/patient may experience a broad range of responses to a treatment (such as the exemplary responses as described herein above).

As used herein, the term "preventing" (or "prevent" or "prevention") refers to the capacity of the activator to minimize or hinder the progression of a disease, condition or disorder in a subject.

As used herein, the term "subject" refers to human and non-human animals, such as veterinary subjects. The term "non-human animal" includes all vertebrates, e.g., mammals and non-mammals, such as non-human primates, mice, rabbits, sheep, dog, cat, horse, cow, chickens, amphibians, and reptiles. In preferred embodiments of the invention, the subject is a human, man or woman of any age or race.

In some embodiments of the invention, the neurological or psychiatric disorder characterized by Kidins220 dysfunction is further characterized by ventriculomegaly i.e., presents ventriculomegaly as one of its symptoms, as defined below.

Ventriculomegaly is a non-specific term describing dilatation of cerebral ventricles. It is the most frequent abnormal central nervous system (CNS) finding detected with prenatal imaging techniques; it is not a disease, but rather a sign that represents a common endpoint of various pathologic processes with different outcomes. Accordingly, the prognosis depends to a large degree on the underlying cause, the coexistence of other anomalies, and the eventual progression, which cannot always be determined with confidence. Ventriculomegaly can be a benign finding, but it can also be associated with aneuploidies, congenital infections, cerebral vascular accidents or haemorrhage, and other fetal cerebral or extracerebral abnormalities, with different implications for long-term neurodevelopmental outcome. Ventriculomegaly is a co-morbidity factor in several psychiatric and neurological disorders, including HCP, schizophrenia, PD and AD.

A neurological or psychiatric disorder characterized by ventriculomegaly refers, in the context of the present invention, to any neurological or psychiatric disorder wherein one of the symptoms is ventriculomegaly.

Neurological and psychiatric disorders characterized by ventriculomegaly include, among others: IS; haemorrhagic stroke; HCP; iNPH; schizophrenia; ASD; spastic paraplegia; intellectual disability; SINO syndrome; ventriculomegaly-cystic kidney disease; and neuromyelitis optica spectrum disorders.

In some embodiments of the invention, the disorder is selected from the group consisting of: HCP; iNPH; IS; schizophrenia; and SINO syndrome.

In some specific embodiments of the invention, the neurological disorder is iNPH.

HCP is a disease associated with cognitive impairment where CSF accumulates provoking ventriculomegaly; notably, it is the pathology that reaches maximal brain ventricles enlargement. There are multiple hydrocephalus forms, including foetal, neonatal, paediatric, and adult-onset presentation, and diverse aetiologies such as genetic and developmental factors, viral infections, co-morbidities, and aging.

HCP may have different causes; congenital hydrocephalus, for example, has been linked to genes that regulate brain growth and development. HCP can also be acquired, mostly from pathological processes that affect ventricular outflow, subarachnoid space function, or cerebral venous compliance. Treatment options include individualised shunt and endoscopic approaches. However, the disease lacks a pharmacological cure.

Dementia is one of the characteristic symptoms of chronic hydrocephalus in adults, where the major form is iNPH.

iNPH is a neurological condition disease occurring in the adult that lacks pharmacological cure and affects gait, balance, cognition and continence. It is characterized by accumulation of CSF and ventricular enlargement, and presents comorbidity with AD, PD and vascular dementia and stroke. The cause of most iNPH cases is unknown, making it difficult to diagnose and understand. Indeed, the clinical symptoms of iNPH may overlap with those of other neurological conditions such as AD and PD, contributing to iNPH misdiagnosis. However, a correct diagnostic is crucial in iNPH, with over 80% patients improving some of the symptoms, but rarely cognition deficits, after treatment with a ventriculoperitoneal shunt. Thus, in addition to the need of pharmacological treatments, there is an urgent need of appropriate tools for differential diagnosis and biomarkers for specific hydrocephalus types or different ventriculomegaly-associated diseases, especially in adults.

In some embodiments of the invention, the neurological or psychiatric disorder characterized by ventriculomegaly is further characterized by dementia.

Dementia is a syndrome, usually of a chronic or progressive nature, in which there is deterioration in cognitive function beyond what might be expected from normal ageing. It affects memory, thinking, orientation, comprehension, calculation, learning capacity, language, and judgement. Dementia results from a variety of diseases and injuries that primarily or secondarily affect the brain. According to recent epidemiological studies, iNPH could account for nearly 10% of dementia cases. Another common cause of dementia is vascular dementia, representing 20% of cases, with IS as the major cause accounting for nearly 80% of cases.

In some embodiments of the invention, the neurological or psychiatric disorder characterized by Kidins220 dysfunction is further characterized by dementia.

In some specific embodiments of the invention, the neurological or psychiatric disorder characterized by Kidins220 dysfunction is further characterized by ventriculomegaly and dementia.

In some specific embodiments of the invention, the neurological disorder is IS.

Stroke is a deadly and debilitating disease, affecting over 15 million people worldwide each year. Ischemic strokes may be caused by either a thrombotic or embolic event that causes a decrease in blood flow to the brain. In a thrombotic event, the blood flow to the brain is obstructed within the blood vessel due to dysfunction within the vessel itself, usually secondary to atherosclerotic disease, arterial dissection, fibromuscular dysplasia, or inflammatory condition. In an embolic event, debris from elsewhere in the body blocks blood flow through the affected vessel. The etiology of stroke affects both prognosis and outcome. Stroke can be accompanied by excitotoxicity and ventricular enlargement known by the term hydrocephalus ex vacuo.

Epilepsy, one of the most common neurological disorders, is characterized by repeated spontaneous seizures with abnormal hyperexcitation and synchronous discharge of neurons that cause excitotoxicity. Whilst several biological factors have been identified as an etiology of epilepsy, including genetic mutation, brain injury, IS, tumor, and aging, the precise cause of epilepsy in most cases is still unknown. One of the hypotheses explaining the pathophysiological mechanism of epilepsy is that the disruption of excitation and inhibition balance (E/I balance) could lead to abnormal excitability of neurons, which may then cause epileptic seizures.

Schizophrenia is a disabling psychiatric disorder that affects 0.7-1% of the population worldwide. When schizophrenia is active, symptoms can include delusions, hallucinations, disorganized speech, trouble with thinking and lack of motivation. However, with treatment, most symptoms of schizophrenia will greatly improve and the likelihood of a recurrence can be diminished.

The disease has a heterogeneous presentation, with disorganized positive and negative symptoms that have different levels of intensity/relevance between individuals and across time in the same individual. However, the most consistent pathological feature in schizophrenic patients is an enlargement of the brain ventricles.

SINO syndrome is a novel, rare genetic neurological disorder with mutations in the *KIDINS220* gene characterized by the association of congenital spastic paraplegia with global developmental delay and intellectual disability, ophthalmologic abnormalities (including nystagmus, reduced visual acuity, or hypermetropia) and obesity. Additional manifestations are brachyplagiocephaly and dysmorphic facial features. Brain imaging may show dilated ventricles, abnormal myelination, and mild generalized atrophy. Additionally, homozygous loss-of-function variants of *KIDINS220* associated with a fetal lethal phenotype with ventriculomegaly and limb contractures have been reported.

In some embodiments of the invention, the neurological or psychiatric disorder characterized by Kidins220 dysfunction is further characterized by excitotoxicity. In some other embodiments of the invention the neurological or psychiatric disorder characterized by Kidins220 dysfunction is further characterized by both ventriculomegaly and excitotoxicity.

A neurological or psychiatric disorder characterized by excitotoxicity refers, in the context of the present invention, to any neurological or psychiatric disorder wherein one of the pathological processes is excitotoxicity.

As stated above, the second most common cause of dementia is vascular dementia, representing 20% of cases. IS, responsible for acute brain injury and neuronal death, is the major cause of vascular dementia, accounting for approximately 80% of all cases. In IS, obstruction of cerebral blood vessels decreases blood flow, compromising glucose and oxygen supply and therefore provoking a dramatic tissue necrotic injury, with massive primary neuronal death at the ischemic core. As a consequence, dying neurons release high amounts of the excitatory neurotransmitter glutamate that can generate a second wave of apoptotic neuronal death in peripheral areas surrounding the nucleus of the infarct thus dramatically exacerbating the initial injury. The primary cause of this secondary neuronal death is known as excitotoxicity.

Excitotoxicity is a critical process in dementia-associated neuropathologies, such as AD, PD and HD; in those pathologies caused by acute brain damage like IS; and in epilepsy and schizophrenia. A massive release of glutamate overstimulates majorly postsynaptic ionotropic NMDARs, overexciting neurons and triggering pro-death cascades. The subsequent excessive influx of Ca2+ leads to the generation of high levels of reactive oxygen species (ROS) and oxidative stress (OS) damage, leading to neuronal death. This condition disturbs ionic homeostasis in neurons and astrocytes and provokes their swelling, synaptic dysfunction and reactive astrogliosis, neuroinflammation, progressive white matter deterioration, destruction and stenosis of microvasculature, disruption of brain barriers and vasogenic edema. Brain edema can be particularly dangerous in ischemia and results from an abnormal accumulation of fluid in the brain with volumetric enlargement, causing brain compression and further damage.

The search of strategies to target excitotoxicity and confer neuroprotection is actively increasing, especially in the stroke field. However, a better knowledge of the molecular mechanisms and the molecular players involved in them is required.

Kidins220 is crucial for neuronal survival and its downregulation contributes to neuronal death after NMDAR overactivation. However, its specific role in excitotoxicity is not completely understood.

AQP4 deficiency decreases glutamate uptake by downregulating the dominant glutamate transporter-1 (GLT-1) in astrocytes and thus could contribute to reach excitotoxic glutamate concentrations. However, the underlying molecular mechanisms remain poorly explored.

As shown for the first time in the present invention, the dramatic drop of AQP4 content at least in ependymal cells and astrocytes in Kidins220 deficient mice suggests their function might be highly compromised. This AQP4 reduction may enhance excitotoxicity.

Additionally, the down-regulation of Kidins220 in Kidins220 deficient mice and the corresponding decrease in the retromer activity may lead to an even higher excitotoxic environment.

As stated above, dementia results from a variety of diseases and injuries that primarily or secondarily affect the brain; some of these diseases are characterized by excitotoxicity. Therefore, in some embodiments of the invention, the neurological or psychiatric disorder characterized by Kidins220 dysfunction and excitotoxicity is further characterized by dementia.

In some specific embodiments of the invention, the neurological or psychiatric disorder characterized by Kidins220 dysfunction, ventriculomegaly and excitotoxicity is further characterized by dementia.

In some embodiments of the invention, the activator of the invention is comprised within a pharmaceutical composition in a therapeutically effective amount. The term "therapeutically effective amount" means the amount of activator of the invention that will elicit the biological or medical response of a tissue, system, animal or human that is being sought by the researcher, veterinarian, medical doctor or other clinician. In the context of the present invention, the biological or medical response to elicit is the treatment and/or prevention of the disorder.

The retromer complex activators of the present invention can be incorporated into pharmaceutical compositions for its administration to the subject. The activators of the invention may conveniently be presented in dosage unit form and may be prepared by any of the methods well known in the art of pharmacy. All methods include the step of bringing the active ingredient into association with the carrier that constitutes one or more accessory ingredients. In general, the pharmaceutical compositions are prepared by uniformly and intimately bringing the active ingredient into association with a liquid carrier or a finely divided solid carrier or both, and then, if necessary, shaping the product into the desired formulation. In the pharmaceutical composition the active compound is included in an amount sufficient to produce the desired effect upon the process or condition of diseases. As used herein, the term "composition" is intended to encompass a product comprising the specified ingredients in the specified amounts, as well as any product which results, directly or indirectly, from combination of the specified ingredients in the specified amounts.

The activator or composition of the present invention may be administered by oral, parenteral (e.g., intramuscular, intraperitoneal, intravenous, ICV, intracisternal injection or infusion, subcutaneous injection, or implant), by inhalation spray, nasal, vaginal, rectal, sublingual or buccal routes of administration and may be formulated, alone or together, in suitable dosage unit formulations containing conventional non-toxic pharmaceutically acceptable carriers, adjuvants and vehicles appropriate for each route of administration.

The pharmaceutical compositions containing the active ingredient may be in a form suitable for oral use, for example, as tablets, troches, lozenges, aqueous or oily suspensions, dispersible powders or granules, emulsions, solutions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients that are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. Oral tablets may also be formulated for immediate release, such as fast melt tablets or wafers, rapid dissolve tablets or fast dissolve films.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

The pharmaceutical compositions may be in the form of a sterile injectable aqueous or oleaginous suspension. This suspension may be formulated according to the known art using those suitable dispersing or wetting agents and suspending agents that have been mentioned above. The sterile injectable preparation may also be a sterile injectable solution or suspension in a non-toxic parenterally acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed, including synthetic mono- or diglycerides. In addition, fatty acids such as oleic acid find use in the preparation of injectables.

The activators or compositions of the invention may also be administered in the form of suppositories for rectal administration of the drug. These compositions can be prepared by mixing the drug with a suitable non-irritating excipient that is solid at ordinary temperatures but liquid at the rectal temperature and will therefore melt in the rectum to release the drug. Such materials are cocoa butter and polyethylene glycols.

In the treatment, prevention, control, amelioration, or reduction of risk of neurological or psychiatric disorders characterized by ventriculomegaly, an appropriate dosage level of the activator of the invention will generally be about 0.01 to 500 mg per kg patient body weight per day, which can be administered in single or multiple doses. For oral administration, the compositions may be provided in the form of tablets containing 1.0 to 1000 milligrams of the active ingredient, particularly 1.0, 5.0, 10.0, 15.0, 20.0, 25.0, 50.0, 75.0, 100.0, 150.0, 200.0, 250.0, 300.0, 400.0, 500.0, 600.0, 750.0, 800.0, 900.0, and 1,000.0 milligrams of the activator for the symptomatic adjustment of the dosage to the patient to be treated.

It will be understood, however, that the specific dose level and frequency of dosage for any particular subject may vary and will depend upon a variety of factors including the activity of the specific compound employed, the metabolic stability and length of action of that compound, the age, body weight, general health, sex, diet, mode and time of administration, rate of excretion, drug combination, the severity of the particular condition, and the subject undergoing therapy.

In some embodiments, the activator or composition of the invention is a SNX27-R activator.

SNX of the SNX17-SNX27-SNX31 subfamily are early endosome-associated proteins that promote the retrieval and recycling of specific cargos. Whereas SNX17 sorts cargos containing NPxY-NxxY motifs such as LRP1 and β1-integrins, the PDZ-domain-containing SNX27 recycles its cargos, including but not limited to, the β2-adrenergic receptor (β2AR) through direct binding to a PDZ ligand located at the carboxy terminus of the receptor.

SNX27, the only SNX that contains a PDZ domain, associates with VPS35, VPS29 and VPS26 ("VPS35-VPS29-VPS26 core") forming the so-called SNX27-retromer, a very selective complex specifically involved in the endosomal-to-plasma membrane recycling of selected cargos, that are degraded at lysosomes upon *SNX27* loss (Steinberg et al. 2013). SNX27-mediated retrieval and recycling of the β2AR is defined by the independent association of SNX27 with the SCAR homologue WASH complex and the SNX27-R, comprising a stable VPS26-VPS29-VPS35 trimer, and a direct SNX27 PDZ-domain-mediated interaction with the VPS26 component of the VPS26-VPS29-VPS35 trimer. Specifically, SNX27 interacts with FAM21, the largest WASH component, which is required for proper recycling of SNX27-R cargos. This interaction restricts SNX27 to an endosomal subdomain, aiding export of cargos to their destinations in an endosomal-to-plasma membrane recycling compartment. Moreover, FAM21 prevents cargos from being transported from endosomes to the Golgi apparatus by controlling Golgi levels of phosphatidylinositol 4-phosphate [PI(4)P], which facilitates a release of retromer cargos around the Golgi.

The SNX27-R plays a major role as a retrieval and recycling hub for a variety of transmembrane proteins, containing specific carboxy terminal PDZ-ligands selective for the binding of the PDZ domain of SNX27, many of which play crucial roles during organism growth and cellular homeostasis. The recycling of endocytosed cargos from early endosomes back to the plasma membrane might be involved in ventriculomegaly development, as suggested by the presence of severe postnatal hydrocephalus in mice lacking *SNX27.* Importantly, retromer impairment also contributes to aberrant endocytic trafficking in various neurodegenerative diseases (Zhang et al. 2018).

The present invention provides unprecedented evidence of the regulatory function of Kidins220 over SNX27-R and ventriculomegaly and of the role of SNX27-R and its retromer-associated proteins in excitotoxicity.

As demonstrated by the inventors, the SNX27-R undergoes a severe process of disorganization in primary cultured neurons under excitotoxic conditions, with some of its components, such as FAM21 and SNX27, being degraded in the presence of high concentrations of NMDA. Furthermore, SNX27 and VPS35 undergo severe changes in subcellular location that are blocked by the NMDARs antagonist DL-AP5, suggesting retromer destabilization or dissociation after NMDAR-mediated excitotoxicity. In addition, DLAP5 hampers the degradation of SNX27R components induced by NMDAR-overstimulation. These destabilization or dissociation and degradation processes are reverted by the activators or compositions of the invention, including the NMDA-induced degradation of SNX27-R cargos Kidins220 and the NMDAR subunit GluN2B, both crucial for neuronal survival. These data demonstrate the potential therapeutic uses of retromer activators in excitotoxicity. Indeed, these pharmacological chaperons show neuroprotection against NMDAR-induced excitotoxicity, as shown herein.

In some embodiments, the activator or composition of the invention is thiophen-2-ylmethyl carbamimidothioate hydrochloride (R33; TPT-172) or thiophene-2,5-diylbis(methylene) dicarbamimidothioate dihydrochloride (R55; TPT-260); or variants thereof.

R55 (CAS# 2076-91-7 (2HCI)) and its analogue R33 (CAS# 32415-42-2 (HCI)) are thiophene thiourea derivatives. Both molecules are pharmacological chaperones that target an area on retromer proteins that will bolster the entire core structure, so that the trimeric Vps35-Vps29-Vps26 core is less prone to degradation. The chaperones increased retromer levels and enhanced retromer-mediated trafficking of cargos (Mecozzi et al. 2014).

The biological activity of R33 has also been tested in a mouse model with plaques and tangles (AD; (Li et al. 2020). Mice treated with the chaperone showed a significant improvement in learning and memory, an elevation of VPS35 levels, and improved synaptic integrity. Additionally, the same animals had a significant decrease in amyloid-beta (Aβ) levels and deposition, reduced tau phosphorylation and less astrocytes activation, demonstrating the enhancement of retromer function by the chaperone.

Similarly, a number of retromer activators derived from R55 have been tested in an Amyotrophic Lateral Sclerosis (ALS), a fatal disease characterized by the degeneration of upper and lower motor neurons (MNs). The best candidate molecule (2a) attenuated locomotion impairment and increased MNs survival (Muzio et al. 2020). Moreover, compound 2a increased VPS35 in inducible pluripotent stem cells (iPSCs) derived MNs and shows brain bioavailability, suggesting the retromer as a valuable druggable target in ALS.

The term "variants thereof" with regard to the retromer stabilizer R55 refers to any variant obtained by replacing the 2,5-disubstituted thiophene scaffold in R55 with a phenyl ring and/or substituting isothioureas with guanylhydrazones, as detailed in Muzio et al. 2020.

In some embodiments of the invention, the retromer complex activator or composition of the invention is selected from the group consisting of: SNX27; VPS35; VPS26; VPS29; FAM21; Kidins220; and AQP4.

SNX27, VPS35, VPS26, and VPS29 are components of the SNX27-R complex; FAM21 is a SNX27-R-associated protein; and Kidins220 and AQP4 are cargo molecules of the SNX27-R complex.

Kidins220 was first described as a SNX27-R cargo targeted to lysosomal degradation in the absence of this PDZ protein (Steinberg et al. 2013). The present invention uncovers the role of Kidins220 as a key determinant in the control of brain water homeostasis, ventricular enlargement and hydrocephalus pathology by different molecular mechanisms that involve the unexpected expression regulation of the SNX27-R components by Kidins220. AQP4, characterized herein as a novel SNX27-R cargo, also appears as a critical player in the cellular processes that contribute to ventriculomegaly.

AQP4 C-terminal amino acids constitute a canonical type I PDZ-L, but differ slightly with those defined as selective for SNX27 PDZ-binding bearing an acidic clamp between P-3 and P-5. AQP4 PDZ-L does not contain acidic residues at both P-3 and P-5, suggesting that selectivity and affinity of SNX27 for its cargos may rely not only on their C-terminal peptide but also on their global context.

The inventors have surprisingly found that both Kidins220 and AQP4 are markedly downregulated at the ventricular ependymal lining of iNPH patients. Additionally, *KIDINS220* deficient mice develop ventriculomegaly accompanied by water dyshomeostasis and loss of AQP4, SNX27 and VPS35 in the brain ventricular ependymal layer and astrocytes. Accordingly, SNX27 silencing decreases AQP4 levels in wild-type astrocytes whereas SNX27 overexpression restores AQP4 content in Kidins220 deficient astrocytes. Therefore, the present invention also uncovers the role of Kidins220 as a key determinant in the control of AQP4 turnover.

In some embodiments of the invention, a retromer complex activator selected from the group consisting of SNX27, VPS35; VPS26; VPS29; FAM21; KIDINS220, and AQP4 is administered to the subject as a nucleic acid.

In some specific embodiments of the invention, the retromer complex activator is administered to the subject as deoxyribonucleic acid (DNA). In other words, the gene encoding the retromer complex activator is administered to the subject.

In some embodiments, the gene encoding the activator of the invention replaces the function of an abnormal or non-functional gene in the subject. In some embodiments, the administration of the gene encoding the activator of the invention leads to a temporary overexpression of the activator.

The concept of gene/DNA delivery and the methods to implement it would be apparent to the skilled person. In some embodiments of the invention, the gene encoding the activator of the invention is delivered as a therapeutic gene expression cassette with the aid of a vector. The cassette comprises a promoter driving gene transcription, the gene encoding the activator of the invention, and a termination signal to end gene transcription.

In some embodiments of the invention, the vector is a viral vector. In some embodiments of the invention, the vector is a retroviral vector. In some specific embodiments of the invention, the vector is a lentiviral vector.

The use of retroviral systems based on these viruses allows for the stable, heritable integration of a specific nucleic acid sequence into the target cell's genome and a theoretically permanent expression of a gene construct, such as an siRNA or protein coding sequence.

In other embodiments of the invention, the vector is an adenoviral vector. In some specific embodiments of the invention, the vector is an adeno-associated viral vector (AAV). In some specific embodiments of the invention, the adeno-associated viral vector is AAV human serotype 10 (AAVrh10) (Park et al. 2017).

AAVs are currently among the most frequently used viral vectors for gene therapy due to their lack of pathogenicity, their persistence, and the many available serotypes. AAV belongs to the parvovirus family and is dependent on co-infection with other viruses, mainly adenoviruses, in order to replicate. Its single-stranded genome contains three genes, Rep (Replication), Cap (Capsid), and aap (Assembly); at least nine gene products are derived from these genes through the use of three promoters, alternative translation start sites, and differential splicing. Importantly, the coding sequences are flanked by inverted terminal repeats (ITRs) that are required for genome replication and packaging.

Recombinant AAV lacking viral DNA traverses the cell membrane and deliver its DNA cargo into the nucleus of a cell. In the absence of Rep proteins, ITR-flanked transgenes encoded within rAAV can form circular concatemers that persist as episomes in the nucleus of transduced cells. Episomal DNA does not integrate into host genomes, resulting eventually in the loss of the transgene and transgene expression. The rate of transgene loss is dependent on the turnover rate of the transduced cell.

In some embodiments, the activator of the invention is SNX27. In some embodiments, the activator of the invention is VPS35. In some embodiments of the invention, the AAVs comprising the gene encoding SNX27 or VPS35 are administered in the brain via intrathecal or intracerebroventricular delivery, leading to the local expression of the gene.

In some embodiments of the invention, two or more complex retromer complex activators selected from the group consisting of SNX27; VPS35; VPS26; VPS29; FAM21; KIDINS220, and AQP4 are administered to the subject as a nucleic acid.

The activators of the present invention may be used in combination with one or more other drugs in the treatment, prevention, control, amelioration, or reduction of risk of neurological or psychiatric disorders characterized by ventriculomegaly in a subject, where the combination of the drugs together is safer or more effective than any of the drugs alone.

Thus, in particular embodiments of the use of the invention, the pharmaceutical composition further comprises at least one compound for the treatment of neurological or psychiatric disorders characterized by Kidins220 dysfuntion in a subject.

In some embodiments, the at least one compound is a compound for the amelioration of secondary effects associated to the neurological or psychiatric disorders characterized by Kidins220 dysfuntion is an analgesic or an anti-inflammatory compound. Examples of analgesic or an anti-inflammatory compound which may be used in conjunction with the activators of the invention include, without being limited to: paracetamol; aspirin; esteroid antimflammatory drugs; dexamethasone; non-esteroid antimflammatory drugs, including but not limited to Ibuprofen, ciclofenac and aceclofenac.

Additional examples of compounds which may be used in conjunction with the activators of the invention include the NMDAR antagonist memantine; thrombolytic compounds; and cell-penetrating peptides.

Such other compound(s)/drug(s) may be administered by a route and in an amount commonly used therefore, simultaneously or sequentially with the activator of the invention. When the activator is used simultaneously with one or more other drugs, a pharmaceutical composition in unit dosage form containing such other drugs and the activator is preferred. However, the combination therapy may also include therapies in which the activator of the invention and one or more other drugs are administered on different overlapping schedules. When used in combination with one or more other active ingredients, the activator of the invention and the other active ingredients may be used in lower doses than when each is used singly. Accordingly, the pharmaceutical compositions of the present invention include those that contain one or more other active ingredients, in addition to an activator.

A second aspect of the present invention relates to a method for screening ("the screening method of the invention") a retromer complex activator for use according to any one of the preceding claims, comprising the following steps: (i) measuring at least one of the following parameters in a non-human animal: a. the ventricular volume of the brain ventricles; and/or b. the levels of expression of at least one gene selected from the group consisting of: *SNX27; VPS35; VPS26*; *VPS29; FAM21; KIDINS220;* and *AQP4* in a biological sample from the non-human animal; (ii) administering a potentially active retromer complex activator for use according to any one of the preceding claims to the non-human animal; (iii) repeating step (i); (iv) evaluating the effect of the potentially active retromer complex activator or composition by: a. comparing the ventricular volumes obtained in (iii)a and (i)a; and/or b. comparing the expression levels obtained in (iii)b and (i)b, and (v) selecting the retromer complex activator that stabilizes the retromer complex, wherein the non-human animal is an animal model with Kidins220 dysfunction; and wherein the retromer complex activator is selected in (v) if the ventricular volumes measured in (iii)a are decreased with respect to the ventricular volumes measured in (i)a, and/or the expression levels of the at least one gene measured in (iii)b are altered with respect to the expression levels measured in (i)b.

Methods for measuring the ventricular volume of the brain ventricles in a non-human animal would be apparent to the skilled person and include, without limitation, measurements in series of Magnetic Resonance Imaging (MRI) images obtained for the animal.

As used herein, a ventricular volume measured in (iii)a and decreased with respect to the ventricular volume measured in (i)a refers to a ventricular volume that is at least: 5% lower, 8% lower, 10% lower, 15% lower, 20% lower, 25% lower, 30% lower, 35% lower, 40% lower, 45% lower, 50% lower, 55% lower, 60% lower, 65% lower, 70% lower, 75% lower, 80% lower, 85% lower, 90% lower, 95% lower, 96% lower, 97% lower, 98% lower, 99% lower, 100% lower, 0.2-fold lower, 0.3-fold lower, 0.4-fold lower, 0.5-fold lower, 1-fold lower, 1.5-fold lower, 2-fold lower, 5-fold lower, 10-fold lower, 20-fold lower, 30-fold lower, 40-fold lower, 50-fold lower, 60-fold lower, 70-fold lower, 80-fold lower, 90-fold lower, 100-fold lower, 1000-fold lower, or more, than the ventricular volume measured in (i)a.

in some embodiments, the ventricular volume measured in (iii)a is at least 0.2-fold lower than the ventricular volume measured in (i)a. In some embodiments, the ventricular volume measured in (iii)a is at least 0.3-fold lower than the ventricular volume measured in (i)a. In some embodiments, the ventricular volume measured in (iii)a is at least 0.4-fold lower than the ventricular volume measured in (i)a.

In some embodiments, the ventricular volume measured in (iii)a is at least 0.5-fold lower than the ventricular volume measured in (i)a.

In some embodiments, the ventricular volume measured in (iii)a is at least 1-fold lower than the ventricular volume measured in (i)a.

In some embodiments, the ventricular volume measured in (iii)a is at least 1.5-fold lower than the ventricular volume measured in (i)a.

In some embodiments, the ventricular volume measured in (iii)a is at least 2-fold lower than the ventricular volume measured in (i)a.

Methods for determining the expression levels of a gene are widely known in the state of the art, and any of them can be used in the context of the present invention. They may comprise measuring the level of cDNA, the level of mRNA, and/or the level of the protein encoded by said gene. By way of illustration and not limitation, the levels of mRNA of said gene can be quantified by means of using conventional methods, for example, methods comprising the amplification of mRNA and the quantification of the product of the amplification of said mRNA, such as electrophoresis and staining, or alternatively, by means of Southern blot and the use of suitable probes, Northern blot and the use of specific probes of mRNA of the gene of interest (gene encoding the protein) or of the corresponding cDNA thereof, S1 nuclease mapping, hybridization, RT-Q-PCR, micro-array and RNA-sequencing, etc. If the quantification of the expression of the gene encoding the protein is going to be carried out from the protein of interest, then the isolated biological sample of the subject must be treated to extract the protein of interest. Methods for extracting or isolating proteins are known by the person skilled in the art and they are commercially available. The levels of gene encoding the protein of interest can be quantified by means of any conventional method that enables said protein to be detected and quantified in a sample of a subject. By way of illustration and not limitation, the levels of said protein can be quantified, for example, by means of the use of antibodies with the capacity to bind specifically to the protein of interest and the subsequent quantification of the complexes formed.

Accordingly, in some embodiments of the invention, the altered expression measured in (iii)a may lead to a decrease or increase in the transcription rate of a gene of about: 5%, 8%, 10%, 15%, 20%, 25%, 30%, 35%, 40%, 45%, 50%, 55%, 60%, 65%, 70%, 75%, 80%, 85%, 90%, 95%, 96%, 97%, 98%, 99%, 100%, 1-fold, 2-fold, 5-fold, 10 fold, 20 fold, 30 fold, 40 fold, 50 fold, 60 fold, 70 fold, 80 fold, 90 fold, 100 fold, 1000-fold, or more, or any value in between these values, when compared to the expression measured in (i)a.

Similarly, in some embodiments, the altered expression measured in (iii)a may lead to a decrease or increase in the amount of mRNA of a gene of about 5%, 8%, 10%, 15%, 20%, 25%, 30%, 35%, 40%, 45%, 50%, 55%, 60%, 65%, 70%, 75%, 80%, 85%, 90%, 95%, 96%, 97%, 98%, 99% or 100%, 1-fold, 2-fold, 5-fold, 10 fold, 20 fold, 30 fold, 40 fold, 50 fold, 60 fold, 70 fold, 80 fold, 90 fold, 100 fold, 1000-fold, or more, or any value in between these values, when compared to the reference sample.

In some embodiments of the invention, the altered expression measured in (iii)a may lead to a decrease or increase in the amount of the polypeptide encoded by the gene of about 5%, 8%, 10%, 15%, 20%, 25%, 30%, 35%, 40%, 45%, 50%, 55%, 60%, 65%, 70%, 75%, 80%, 85%, 90%, 95%, 96%, 97%, 98%, 99% or 100%, 1-fold, 2-fold, 5-fold, 10 fold, 20 fold, 30 fold, 40 fold, 50 fold, 60 fold, 70 fold, 80 fold, 90 fold, 100 fold, 1000-fold, or more, or any value in between these values, when compared to the reference sample.

As would be apparent to the person skilled in the art, any altered expression of the gene at protein level may or may not be associated with an altered expression of the gene at the mRNA level.

The levels of expression may be determined using any type of biological sample from the subject. As used herein, the term "biological sample" refers to any material comprising a nucleic acid and/or a polypeptide of interest. Examples of biological samples include, but without limiting to, a biopsy sample, a tissue (e.g., brain tissue), cell or fluid (e.g., serum, saliva, semen, sputum, cerebral spinal fluid (CSF), tears, mucus, sweat, brain extracts and the like) and blood (e.g., PBMCs (peripheral blood-derived mononuclear cells) such as neutrophils, monocytes).

In some embodiments of the invention, the levels of expression of the protein of interest are determined in a blood or CSF sample from the subject.

According to the method for screening of the invention, the retromer complex activator is selected if the ventricular volumes measured in (iii)a are decreased with respect to the ventricular volumes measured in (i)a, and/or the expression levels of the at least one gene measured in (iii)b are altered with respect to the expression levels measured in (i)b.

Within the context of the present invention, the term "altered" refers to either an increased or a decreased expression level of the at least one gene measured in (iii)b with respect to the expression levels measured in (i)b. By way of non-limiting example, the protein encoded by the at least one gene may accumulate in the brain in certain diseases, i.e., the gene expression level in the brain measured in (iii)b is increased with respect to the expression level measured in (i)b. However, the expression levels of the same gene in the same disease measured in (iii)b may be decreased in the CSF or any other type of biological sample with respect to the expression level measured in (i)b.

By way of non-limiting illustration, levels of beta amyloid 1-42 peptide are increased in brain samples obtained from subjects suffering from AD with respect to levels measured in (i)b; however, beta amyloid 1-42 levels are decreased in CSF. Conversely, the expression level of the phosphorylated forms of the protein tau (phospho-tau) are increased in both brain and CSF samples obtained from subjects suffering from AD with respect to the levels measured in (i)b.

In certain embodiments of the invention, the expression levels of the genes of interest measured in (iii)b are altered both in the brain and the CSF of Kidins220 deficient animal with respect to the expression level measured in (i)b.

In certain embodiments of the invention, the expression levels of the genes of interest measured in (iii)b are decreased both in the brain and the CSF of Kidins220 deficient animal with respect to the expression level measured in (i)b.

In some embodiments of the invention, the animal model with Kidins220 dysfunction is a rodent.

The use of rodents as non-human animal models is well known in the state of the art. Rats and mice have been the leading model organisms used in biomedical research for well over a century, with mice rapidly overtaking rats as the major model of choice in biological research in the last years. This shift may be related to the availability of techniques for genetic manipulation of mice, including the generation of knockout mice.

In some specific embodiments of the invention, the rodent is a Kidins220 floxed (Kidins220^{f/f}) mouse (Cesca et al. 2012).

A high number of knockout mouse strains show a prenatal lethality phenotype. To study the gene functions in adult mice, conditional knockout, which allows for precise control of genetic modifications in specific tissues and at specific stages, is necessary. The most commonly-used system for conditional knockout is Cre/lox, which uses a site-specific Cre recombinase and its target sequence lox with unique 34-bp sequences. In this system, a region of interest flanked by two lox sites (floxed) is deleted or inverted by Cre-mediated recombination, leading to gene knockout only in a Cre-expressing cell. In general, Cre/lox mice are generated by mating a Cre-driver mouse with a flox mouse.

Traditionally, flox mice have been obtained by gene targeting in embryonic stem cells followed by production of germline chimeric mice. Recently, genome editing using direct injection of engineered endonucleases or RNA-guided nucleases into zygotes has greatly accelerated the production of gene-modified animals. The most popular system, clustered regularly interspaced short palindromic repeats (CRISPR)/CRISPR-associated (Cas), is based on RNA-guided nucleases.

The Kidins220^{f/f} mice constitute a hypomorphic mouse model of Kidins220 dysfunction showing various degrees of Kidins220 downregulation that positively correlate with ventriculomegaly penetrance. The majority of Kidins220^{f/f} born mice reached adulthood without showing apparent external evidence of overt hydrocephalus, suggesting that ventricle enlargement progresses slowly in these animals. As shown herein, while the brain of Kidins220^{-/-} embryos presents a pronounced enlargement of LVs, TV and FV, 2-month-old Kidins220^{f/f} animals only show enlargement of the LVs and TV. This suggests an incipient nature of hydrocephalus in 2-month-old Kidins220^{f/f}, which might develop progressively with aging.

The Kidins220^{f/f} mice thus represent an attractive model to study SINO syndrome, Kidins220-linked schizophrenia and other neuropathologies accompanied by ventriculomegaly such as foetal/perinatal to adulthood onset forms of HCP.

A third aspect of the present invention relates to an *in vitro* method for the diagnosis of a neurological or psychiatric disorder characterized by Kidins220 dysfunction in a human subject ("the diagnosis method of the invention"), comprising: (i) measuring the expression levels of at least one gene selected from the group consisting of: *SNX27; VPS35; VPS26*; *VPS29; FAM21; KIDINS220;* and *AQP4* in a biological sample from the human subject, and (ii) comparing the expression levels measured in (i) with reference levels measured in a biological sample from a control human subject, wherein if the expression levels of the at least one gene listed in (i) are altered with respect to the reference level, then the human subject is suffering from a neurological or psychiatric disorder characterized by Kidins220 dysfunction.

As stated elsewhere in this document, the neurological or psychiatric disorder characterized by Kidins220 dysfunction may be further characterized by ventriculomegaly and/or excitotoxicity. Therefore, the diagnosis method of the invention may be used for the diagnosis of a neurological or psychiatric disorder in a subject where ventriculomegaly and/or excitotoxicity have been detected, and an associated Kidins220 dysfunction is suspected.

The term "reference level" refers to the expression levels of the gene of interest in a biological sample from a subject other than the subject being diagnosed with the method of the invention. Specifically, it refers to the expression levels of the gene of interest in a biological sample from a control subject. As defined above, a control subject is a subject not suffering from any cerebrovascular, neurological or psychiatric disorders ("control subject/s"). The reference level may be calculated as an average value from reference levels of the gene of interest in *n* control subjects, wherein *n* is a whole number equal to or higher than 2.

All the relevant terms have been explained above in the present description, and these explanations, as well as its particular embodiments, are applicable to the present inventive aspect.

In some embodiments, the subject suffering from the neurological or psychiatric disorder is susceptible to receive a therapy based on the activator or composition of the invention.

As described above, if the expression levels of the at least one gene listed in (i) are altered with respect to the reference expression levels measured in a biological sample from a control human subject, then the human subject may be susceptible to receive a therapy based on the activator or composition of the invention.

In some embodiments of the invention, the neurological or psychiatric disorder is selected from the group consisting of: IS; epilepsy; HCP; iNPH; schizophrenia; and SINO syndrome.

In some specific embodiments of the invention, the neurological or psychiatric disorder is iNPH.

In some specific embodiments of the invention, the neurological or psychiatric disorder is IS.

In further inventive aspects, the present invention also encompasses a method of treating or preventing neurological or psychiatric disorders characterized by Kidins220 dysfunction in a subject comprising the use of the activator of the composition of the invention. It also encompasses the use of the activator or composition of the invention as described herein in the manufacture of a medicament for the treatment or prevention of neurological or psychiatric disorders characterized by Kidins220 dysfunction in a subject. All the preferred embodiments and explanations of the terms used in these inventive aspects have been explained above.

As stated elsewhere in this document, the neurological or psychiatric disorder characterized by Kidins220 dysfunction may be further characterized by ventriculomegaly and/or excitotoxicity. Therefore, the further inventive aspects of the invention also comprise: (i) a method of treating or preventing neurological or psychiatric disorders in a subject where ventriculomegaly and/or excitotoxicity have been detected, and an associated Kidins220 dysfunction is suspected, and wherein the method of treating or preventing said disorder comprises the use of the activator of the composition of the invention; and (ii) the use of the activator or composition of the invention as described herein in the manufacture of a medicament for the treatment or prevention of neurological or psychiatric disorders initially characterized by ventriculomegaly and/or excitotoxicity in a subject.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### BRIEF DESCRIPTION OF THE DRAWINGS

**Figure 1****.** Ventriculomegaly in Kidins220 hypomorphic mice.
**Figure 2****.** Decreased AQP4 levels at the ependymal barrier of Kidins220 hypomorphic mice and human iNPH.
**Figure 3****.** AQP4 downregulation in Kidins220-deficient astrocytes is rescued by inhibition of lysosomal degradation.
**Figure 4****.** Joint regulation of the SNX27-retromer and its novel cargo AQP4 by Kidins220.
**Figure 5****.** AQP4 downregulation by Kidins220 deficiency is mediated by SNX27.
**Figure 6****.** Effects of the pharmacological chaperone R55 on protein levels of the retromer complex and its cargos in astrocytes.
**Figure 7****.** Excitotoxicity induced by NMDAR-overstimulation provokes degradation and dissociation of SNX27-retromer components in primary neuronal cultures.
**Figure 8****.** The pharmacological chaperone R55 prevents NMDAR-mediated excitotoxic dissociation and degradation of SNX27-retromer components and cargos in primary neuronal cultures.
**Figure 9****.** Neuroprotection from NMDAR-induced excitotoxicity by the pharmacological chaperone R55 in primary neuronal cultures.

### EXAMPLES

### Materials and methods

Human brain samples. Brain samples from iNPH patients (n = 6) and non-normotensive hydrocephalus patients (n=3), and control subjects (n = 8) were obtained from the Institute of Neuropathology Brain Bank (IDIBELL, Hospitalet de Llobregat, Spain), tissue Biobank from Hospital Clinic de Barcelona and Institute d'Investigacions Biomediques August Pi I Sunyer (HCB-IDIBAPS Biobank), and CIEN Foundation Tissue Bank for Neurological Research (BT-CIEN) following Spanish legislation and local Ethics Committee guidelines and processed. All participants gave their written consent, and the study was approved by the local Ethics Committee following the ethical standards recommended by the Helsinki Declaration.

Experimental animals. Kidins220^{f/f} mice and Kidins220-/- mice in C57BL/6J background had been previously generated in G. Schiavo laboratory (Cesca et al. 2012). Embryos from 19-day-old Wistar rat were used to prepare cortical neuronal cultures. All animals were produced and housed at the animal care facility at Instituto de Investigaciones Biomédicas "Alberto Sols" (IIBM, CSIC-UAM, Madrid, Spain) and maintained under 12/12 h light-dark cycle and with access to food and water ad libitum in a temperature-controlled environment. Overall animal health was assessed by daily inspection for signs of discomfort, weight loss, or changes in behaviour, mobility, and feeding or drinking habits. Procedures involving animals had been approved by Institutional (IIBM and CSIC) and local Ethical Committees, and were conformed to the appropriate national legislations (RD 53/2013) and the guidelines of the European Commission for the accommodation and care of laboratory animals (revised in Appendix A of the Council of Europe Convention ETS123).

Magnetic resonance imaging. MRI studies were performed in the High Field MRI and Spectroscopy facility (IIBM, CSIC-UAM) using a Bruker Biospect 7.0-T horizontal-bore system (Bruker Medical Gmbh, Ettlingen, Germany), equipped with a 1H selective birdcage resonator of 23 mm and a Bruker gradient insert with 90 mm of diameter (maximum intensity 36 G/cm). All data were acquired using a Hewlett-Packard console running Paravision software (Bruker Medical Gmbh) operating on a Linux platform. Anaesthesia was initiated by inhalation of oxygen (1 L/min) containing 3% isoflurane and maintained during the experiment employing a mask and 2% isoflurane in O2. Animal temperature was maintained at approx. 37°C with a heating blanket. The respiratory rate of the animals was monitored using a Biotrig physiological monitor (SA Instruments, Stony Brook, NY) and the breathing rate was maintained in 60 +/- 40 bpm. T2-weighted (T2-W) spin-echo anatomical images were acquired with a rapid acquisition with relaxation enhancement (RARE) sequence in axial orientations and the following parameters: TR = 2500 ms, TE = 44 ms, RARE factor = 8, Av = 6, FOV = 2.3 cm, acquisition matrix = 256 x 256, slice thickness = 1.00 mm without gap and number of slices = 14. To calculate the ventricular volume of each ventricle in mm3, the ventricular area was measured in all series of images obtained in MRI-T2W for each animal. Diffusion weighted images (DWI) were acquired with the following parameters: TR = 3500 ms, TE = 27 ms, Av =3, 3 gradient direction, diffusion gradient duration = 3 ms, diffusion gradient separation = 18 ms, b factors = 200, 400 and 1000s/mm2, FOV: 2.5cm, acquisition matrix = 128 x 128, slice thickness: 1.5mm without gap and number of slices 7. Apparent Diffusion Coefficient (ADC) maps were calculated with a homemade software application written in Matlab (R2007a). The values were extracted from maps using the region of interest (ROI) with the Image J software (Wayne Rasband, NIH, Bethesda, MD, USA) as described above.

Plasmids. Lentiviral vectors containing shRNAs to interfere mouse Kidins220 (shKidins220) and mouse Snx27 (shSNX27-1, shSNX27-2, shSNX27-3) expression were generated by cloning sh-oligonucleotides into the Hpal and Xhol sites of pLentiLox3.7 (pLL3.7; see detailed sequences in Supplementary Table 3). Control shRNA vector (shC) was constructed by introducing oligonucleotides that do not match any known mice transcript. The integrity of these novel constructs was verified by sequencing. SNX27 full length (SNX27-FL), ΔPDZ-SNX27 and mCherry-SNX27 expression vectors were kindly provided by Peter Cullen (University of Bristol, UK). SNX27-FL and ΔPDZ-SNX27 cDNA sequences were subcloned into Hindlll and Apal sites of pEGFP-C3 to generate GFP-SNX27-FL and GFP-ΔPDZ-SNX27 plasmids. GFP-M23-AQP4 expression vector was provided by Mahmood Amiry-Moghaddam (University of Oslo, Norway).

Cell culture, treatment and transfection of primary cortical astrocytes, neurons and HEK293T cells. Mouse cortical astrocytes were prepared from cerebral cortices of 0 to 2-day-old mice pups according to standard methods. Cells seeded on plates or glass coverslips coated with poly-L-lysine (10 µg/ml) and laminin (4 µg/ml) at a density of 5 x 10⁴ cells/cm2 in complete DMEM-F12. After 2 additional days (DIV14-16) astrocytes were treated with vehicle (DMSO), 100 nM bafilomycin A1 (Sigma Co.; St. Louis, MO, USA) for 16 h or 10 µM R55 (MedKoo Biosciences, Inc.; Morrisville, NC, USA) for 48 h. Neuronal cultures were prepared from cerebral cortex of 19-day-old Wistar rat embryos, as we have previously described (Lopez-Menendez et al. 2019). Neurons were used after 10 days in vitro (DIV) for immunofluorescence and DIV14 for biochemical assays. Neurons were pretreated or treated for different times as indicated: 50 µM NMDA and 10 µM glycine (Sigma Co.; St. Louis, MO, USA); 200 µM DL-AP5 (Tocris; Bristol, UK); 5 µM R55. Excitotoxicity was induced by treatment with the NMDAR coagonists NMDA and glycine (referred as NMDA treatment). DL-AP5 or R55 were added 1 h or 48 h before NMDA treatment, respectively. HEK293T cells were cultured and transfected by standard procedures. HEK293T cells and DIV12-14 primary astrocytes were seeded at 50-60% confluence. Two days later cells were transfected in Opti-MEM by using 0.7 µl of Lipofectamine 2000^{®} reagent (ThermoFisher Scientific) and 0.7 µg of DNA per 24 multi-well plate, according to the manufacturer's specifications. Cells were fixed and processed for immunofluorescence or washed with PBS to prepare protein extracts for immunoprecipitation experiments.

Assessment of neuronal viability in primary cultures. Measurement of neuronal survival by MTT reduction assay (Sigma Co.) was performed as specified by the manufacturer.

Lentiviral production and transduction of astrocytic cultures. Lentiviral suspensions were prepared in HEK293T cells. Briefly, HEK293T were transfected with lentiviral and packaging vectors using Lipofectamine 2000^{®} reagent and OPTI-MEM media (ThermoFisher Scientific) for 4 h following the manufacturer instructions. Medium was then changed to Iscove's Modified Dulbecco's Medium (IMDM) complemented with 5% FBS, 5% horse serum, 22.2 mM glucose, 0.1 mM Glutamax-I, penicillin (100 U/ml), and streptomycin (100 U/ml) (ThermoFisher Scientific). Supernatant was collected after 48 h. For concentration, the viral suspension was first filtered using a Steriflip-HV 0.45-µm filter unit (Millipore, Billerica, MA, USA) and ultracentrifuged at 20,000 rpm for 2 h at 4°C in a SW28 Beckman Coulter rotor. Viral pellets were resuspended overnight at 4°C in PBS. DIV12-14 cultured astrocytes were transduced with concentrated lentiviral suspensions (107-108 pfu/ml) directly added to the growing media for 7 days.

Water Permeability and Cell-Volume Measurements in cultured astrocytes. Cell-volume changes in WT and Kidins220^{f/f} astrocytes were analysed by the calcein-quenching fluorescence method. DIV12-14 cultured astrocytes were grown on glass bottom dishes coated with poly-L-lysine and laminin for 2 days before in vivo confocal microscopy analysis. Cells were maintained with the appropriate saline buffer at room temperature and loaded with calcein (ThermoFisher Scientific) for 30 min at 37ºC in the incubator. Calcein dye was excited at 495 nm, and emission was detected at 525 nm. For a steady baseline recording and defining regions of interest (ROIs) around individual cells to collect calcein photometric data, cultures were maintained for 60 s in control saline. The initial average resting fluorescence, expressed in fluorescence units (F), of an individual cell during 60s perfusion in control isotonic medium (300 mOsm) was obtained and normalized to 1.00 (Ft/Ft0) to measure volume-regulatory behaviour using ImageJ software (Wayne Rasband, NIH, Bethesda, MD, USA). The hypotonic challenge was rapidly applied by adding water to reach hypotonic solutions (150 mOsm; ΔOsm = 150 mOsm and 75 mOsm; ΔOsm= 225 mOsm), causing cell swelling that was registered as a variation in calcein fluorescence signal at each time point (ΔFt). Means of ΔFt values recorded at 65 s and 170 s after hypotonic challenge were calculated and compared in order to establish water permeability relative to each condition. Maximum change in fluorescence defined as variation of fluorescence (ΔFtmax) corresponds to the maximum change in the volume after hypotonic challenge exposure. This challenge initiated a regulated volume decrease (RVD) leading to fluorescence recovery toward the control level. The regulatory volume changes were calculated by the mean of Δ(Ft300-Ftmax170s)/Ftmax170s, being Ftmax the maximum level of fluorescence corresponding to maximum change in volume for each condition.

Commercial antibodies. Rabbit polyclonal antibodies were: Kidins220 (Generated at T. Iglesias laboratory; (Lopez-Menendez et al. 2019)), AQP4 (Sigma-Aldrich, St Louis, MO, USA), β-catenin (Cell Signalling Technology, Beverly, MA, USA), GFP (ThermoFisher Scientific), SNX17 (Proteintech, Manchester, UK) and FAM21 (EMD Millipore Co, Burlington, MA, USA). Goat polyclonals were: VPS35 and GFAP (Abcam, Cambridge, UK). Chicken polyclonal was microtubule-associated protein 2 (MAP2), (Abcam, Cambridge, UK). Mouse monoclonals were: β-actin, α-tubulin, S100β, γ-tubulin (Sigma-Aldrich), GFAP, FoxJ1 (ThermoFisher Scientific), Kidins220, SNX27 (Abcam), Rab5 (Synaptic Systems, Goettingen, Germany), NSE (ICN Biomedicals, Costa Mesa, CA, USA), GluN2B (BD Biosciences, San Jose, CA, USA) and non-erythroid spectrin (Chemicon, Temacula, CA, USA). Horseradish peroxidase-conjugated anti-rabbit and anti-mouse secondary antibodies were from Santa Cruz Biotechnology and Alexa-Fluor-488, -555, and -647 conjugated anti-rabbit, anti-mouse and anti-goat antibodies were from ThermoFisher Scientific.

Preparation of protein extracts, immunoprecipitation, and immunoblot analysis. Protein extracts from mice tissue, primary cell cultures or HEK293T cells were prepared in RIPA by standard methods. Briefly, cultured cells were lysed in RIPA buffer (25 mM Tris-HCI, pH 7.6, 1% Triton X-100, 0.5% sodium deoxycholate, 0.1% SDS, 150 mM NaCI) with protease and phosphatase inhibitors for 30 min at 4 °C. Lysates were centrifuged for 30 min at 14,000 rpm at 4°C, and resulting supernatant was considered the total lysate soluble fraction. Different mouse tissues were dissected, frozen in dry ice and homogenized in RIPA buffer as above using potter and Polytron homogenizers. For immunoprecipitation, total lysates were incubated with anti-VPS35 or anti-GFP antibody for 4 h at 4°C, before binding immunocomplexes to protein A sepharose beads for 1 h at 4°C. Beads were washed four times with RIPA buffer before adding Laemli sample buffer. Note that sample boiling was avoided to detect properly AQP4 band by immunoblots. Equal amounts of total lysates or immunoprecipitates were resolved in SDS-PAGE and analysed by immunoblot. Membranes were incubated with different primary and secondary antibodies and immunoreactive bands were detected by ECL (PerkinElmer, Waltham, MA USA). Immunoblot images have been cropped for presentation.

Immunofluorescence of cultured astrocytes and neurons. Primary astrocytes or neurons grown on coverslips coated with poly-L-lysine and laminin were fixed with 4% PFA with 4% sucrose, permeabilised with 0.1% Triton X-100 in PBS for 5 min and then incubated in blocking solution (5% donkey serum in PBS) for 30 min at room temperature. Coverslips were then incubated for 1 h at room temperature with the appropriate primary antibody in blocking solution followed by the corresponding secondary antibody. Nuclei were stained with DAPI and coverslips were mounted with Fluoromount-G medium (ThermoFisher Scientific).

Immunohistochemistry of mouse and human brain samples. Mice were anesthetized by an i.p. injection of pentobarbital and then transcardially perfused with 4% PFA. Brains were removed from mice skull and post-fixed in 4% PFA overnight and cryoprotected in 30% sucrose in PBS for 48 h before freezing. Complete series of 30 µm-thick coronal sections were obtained by cryostat (Leica Microsystems, Germany). For immunofluorescence floating sections were incubated in blocking solution (5% goat serum, 1% BSA and 0.1% Triton X-100 in 0.1M PBS) for 1 h and incubated in blocking buffer with the appropriate primary antibodies at 4ºC overnight and subsequently incubated 2 h at 4ºC with secondary antibodies. DAPI was used for nuclear staining before mounting slices with Fluoromount-G medium (ThermoFisher Scientific). Paraffin embedded human post-mortem brain 15-µm sections were processed, treated with EnVision FLEX (Dako, K8004, Denmark) for antigen retrieval during 20 min at 90ºC and incubated in blocking solution (10% donkey serum and 0.2% Triton X-100 in PBS) for 1 h. For double immunofluorescence analysis, sections were incubated with primary antibodies for 2 days in blocking solution followed by washes in PBS and incubation with secondary antibodies in the same solution. Then, slices were rinsed in PBS and nuclei were stained with DAPI. Sections were treated with a saturated solution of Sudan Black B (Sigma-Aldrich) for 15 min to block autofluorescence of lipofuscin granules. Sections were later washed and mounted with Prolong medium (ThermoFisher Scientific). Specific regions of interest (ROI) boundaries were defined at the ependymal cells lining lateral ventricles, whose nuclei were stained with DAPI, and Kidins220 or AQP4 immunofluorescence intensity signal was quantified. Quantification studies were performed at 9 different ROIs defined at the ependymal barrier area of each individual.

Image acquisition. Confocal microscopy images were acquired using plan-apochromatic objectives in an inverted Zeiss LSM 710 laser-scanning microscope (Zeiss, Germany). To avoid crosstalk between channels, sequential scanning mode was used. All images shown correspond to maximum projection of serial sections, except those used for colocalization analysis. Pictures were processed with Zen 2009 (Carl Zeiss MicroImaging), Adobe Photoshop CS (Adobe Systems Inc.), and ImageJ 1.47d (NIH) software.

Quantitative and statistical analysis. Immunoblot signals were quantified by densitometric analysis with ImageJ 1.47d software (NIH) and normalized using β-actin, α-tubulin or GFAP band signal intensities. Data were expressed relative to values obtained in their respective untreated controls or WT mice or astrocytes. For immunofluorescence quantitative analysis of mice and human brain samples, fluorescence intensity of specific antibodies was measured at the ependymal barrier of lateral ventricles using ImageJ 1.47d software (NIH). Specific ROIs were defined in each brain section at the ependymal barrier boundaries, identified by DAPI staining of ependymal cell nuclei. In those regions, fluorescence signal above a certain threshold, stablished as background, was quantified. In the case of human brain necropsies, nine randomly selected ROIs were used for the analysis. For murine samples, three independent sections per animal were used, each containing 3 ROIs. In both cases, results were expressed as the average fluorescence intensity, and relative to values detected in control samples, arbitrarily assigned a value of 100. In cultured astrocytes fluorescence intensity of specific antibodies was measured in 60-80 GFAP+ cells for each condition or 40-80 GFP+ cells in those astrocytic cultures transfected or transduced with GFP-expressing plasmids. Analyses of significant differences between means were carried out using unpaired Student's t test, one-way or two-way ANOVA tests with Tukey multiple comparison test, when appropriated according to the data distribution. Variance was comparable between groups in experiments described throughout the manuscript. A Grubbs' test, also called the ESD method (extreme studentized deviate) was performed to determine whether one of the samples or animals in each group was a significant outlier from the rest, with a significant level of α=0.05. No statistical method was used to predetermine sample size. Sample sizes were based on previously published experiments by others and us, and on our own unpublished data. The experiments were not randomized. The investigators were not blinded to allocation during experiments or outcome assessment. Results are shown as mean ± s.e.m.. Statistical significance was assigned at *P < 0.05, **P < 0.01 and ***P < 0.001.

### Results

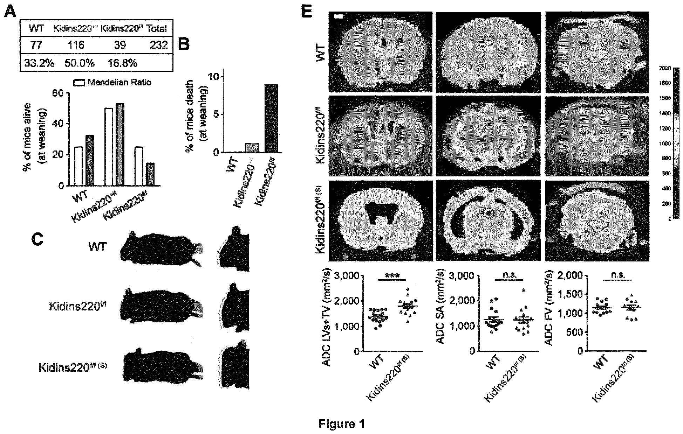

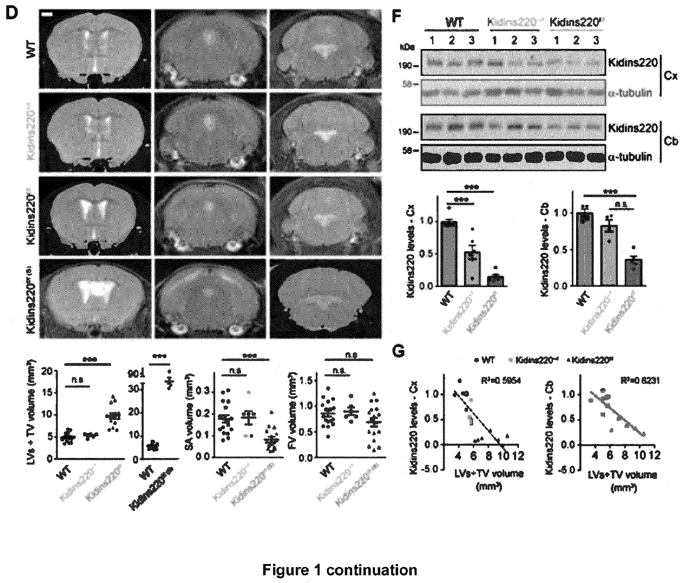

Kidins220 deficient mice present ventriculomegaly. Because Kidins220 complete ablation produces perinatal lethality, we created Kidins220-floxed mice to generate mouse models with conditional deletion in different tissues and cells to study this molecule function in adulthood (Cesca et al. 2012). We noticed that homozygous Kidins220-floxed mice (Kidins220f/f) were not present in the offspring of heterozygous (Kidins220+/f) crosses at Mendelian ratio, with a greater lethality at weaning (Fig. 1a, b). It was also visually apparent that a low percentage of Kidins220^{f/f} mice presented encephalomegaly (17.4%), referred to as 'severe' (S) (Kidins220f/f(S)), suggesting possible brain ventricular enlargement and hydrocephalus (Fig. 1c). MRI analysis confirmed the development of ventriculomegaly to various extents, from mild to severe, in Kidins220^{f/f} mice (Fig. 1d). Volumetric quantification from T2-weighted (T2-W) images rendered increments in the volume of lateral ventricles (LVs) and third ventricle (TV) of Kidins220^{f/f} animals compared to wild type (WT) or Kidins220^{+/f} littermates (Fig. 1d). While no substantial changes were detected in the fourth ventricle (FV), the aqueduct of Sylvius (SA) volume was decreased in Kidins220^{f/f} mice (Fig. 1d). Using diffusion-weighted imaging MRI, we also calculated apparent diffusion coefficient (ADC) values as a measurement of the magnitude of diffusion of water molecules in the ventricles and SA. ADC values were elevated in LVs and TV from Kidins220^{f/f} mice relative to WT animals suggesting an imbalance in the circulation of CSF (Fig. 1e).

The generation of Kidins220^{f/f} animals involved genetic manipulations that integrated Kidins220 cDNA within exon 16 of the mouse gene (see details in Cesca et al. 2012). We hypothesized that this strategy might potentially cause a deficiency in Kidins220 expression. To test this hypothesis, we analysed Kidins220 levels in various brain regions. Immunoblot analysis showed that the cortex and cerebellum of Kidins220^{f/f} mice exhibited decreased amounts of Kidins220 compared to WT animals (Fig. 1f). The cortex of Kidins220^{+/f}, but not the cerebellum, also presented substantially reduced levels of this protein, indicating that some brain regions are potentially more susceptible to the interference induced by the transgenic modification. Importantly, we found an inverse correlation between Kidins220 levels and LVs and TV ventricular volume in WT, Kidins220^{+/f}, and Kidins220^{f/f} mice (Fig. 1g), suggesting that Kidins220 deficiency and brain ventricle enlargement are strongly linked. In addition to brain, extracts obtained from several tissues showed decreased levels of Kidins220 to various degrees between WT, Kidins220^{+/f}, and Kidins220f/f mice (not shown). Together, these data indicate that Kidins220^{f/f} mice constitute a hypomorphic model with marked deficiency of Kidins220 in several tissues, supporting the notion that the decrease of Kidins220 brain levels is linked to ventriculomegaly and hydrocephalus development.

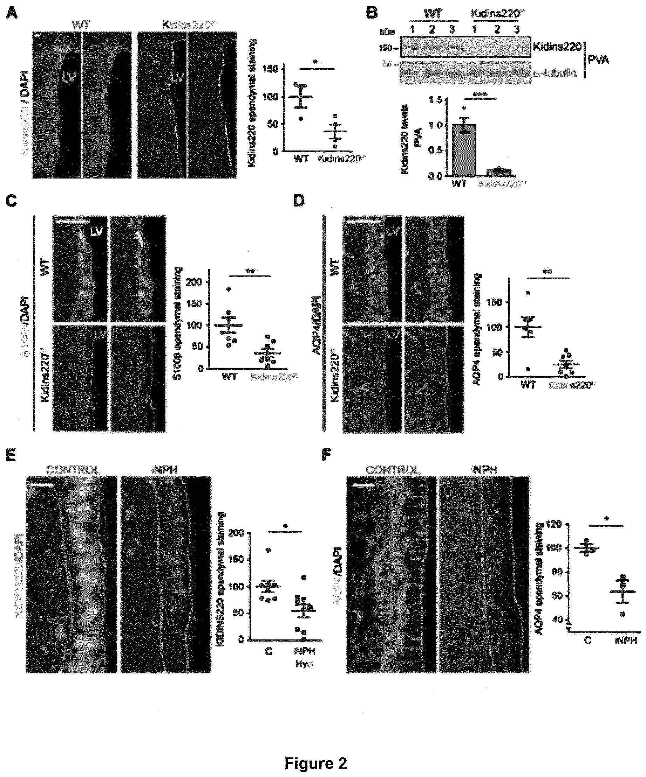

Loss of Kidins220 and AQP4 at the ventricular ependyma of Kidins220 hydrocephalus mice and human iNPH patients. Due to the importance of the ventricular ependyma in brain water homeostasis and congenital hydrocephalus, we examined the presence of Kidins220 in this specialised tissue by using a recently generated antibody (Lopez-Menendez et al. 2019). Confocal microscopy analysis showed higher ependymal Kidins220 expression in the lateral ventricles of WT mice compared to Kidins220^{f/f} mice (Fig. 2a). Accordingly, Kidins220 levels were significantly lowered in extracts prepared from the lateral periventricular area (PVA) of Kidins220^{f/f} animals (Fig. 2b).

The ependymal barrier lines cerebral ventricles and is formed by multiciliated ependymocytes that beat ventricular CSF synchronously to drive its unidirectional flow across ventricles. Different types of congenital hydrocephalus present developmental defects in ependymocytes differentiation, ciliogenesis and maturation, or denudation of the ependymal barrier. We therefore examined the morphological features of the ependymal cells in LVs of 2-month-old Kidins220^{f/f} mice by scanning electron microscopy, finding no apparent macroscopic changes in their cilia compared to WT animals (not shown). In addition, organization and integrity of the ependymal barrier in Kidins220 deficient mice was largely unaltered, as assessed by whole-mount preparation immunostaining and localization of β-catenin at the intercellular contacts and γ-tubulin at the base of the cilia (not shown).

Although the ultrastructural appearance and planar polarity of the ependymal barrier in Kidins220^{f/f} animals was normal, ependymal cells lacked expression of S100 calcium-binding protein β (S100β), a protein expressed in ependymocytes and astrocytes (Fig. 2c). We also examined levels of AQP4 in this area as this channel is also expressed in ependymal cells and plays a critical role in brain water homeostasis and ependyma function (Papadopoulos et al. 2013). Kidins220^{f/f} mice exhibited a striking downregulation of AQP4 in ependymal cells of LVs compared to WT animals (Fig. 2d).

Next, we examined potential decreases of KIDINS220 and AQP4 at the ventricular ependyma in iNPH patients compared to control donors. We found that ependymal cells were positive for KIDINS220 signal in brain necropsies from control individuals and that this staining diminished significantly in samples from equivalent brain regions from iNPH patients (Fig. 2e). Samples from adult non-normotensive hydrocephalus (Hyd) patients similarly presented a fainter KIDINS220 ependymal barrier signal than aged-matched controls (Fig. 2e). Notably, AQP4 immunofluorescence analysis also showed a sharp signal drop in samples from iNPH patients (Fig. 2f). This is the first evidence of an ependymal deficit of AQP4 and KIDINS220 in iNPH patients, supporting the involvement of the ependymal barrier and these two molecules in the development of adult chronic hydrocephalus.

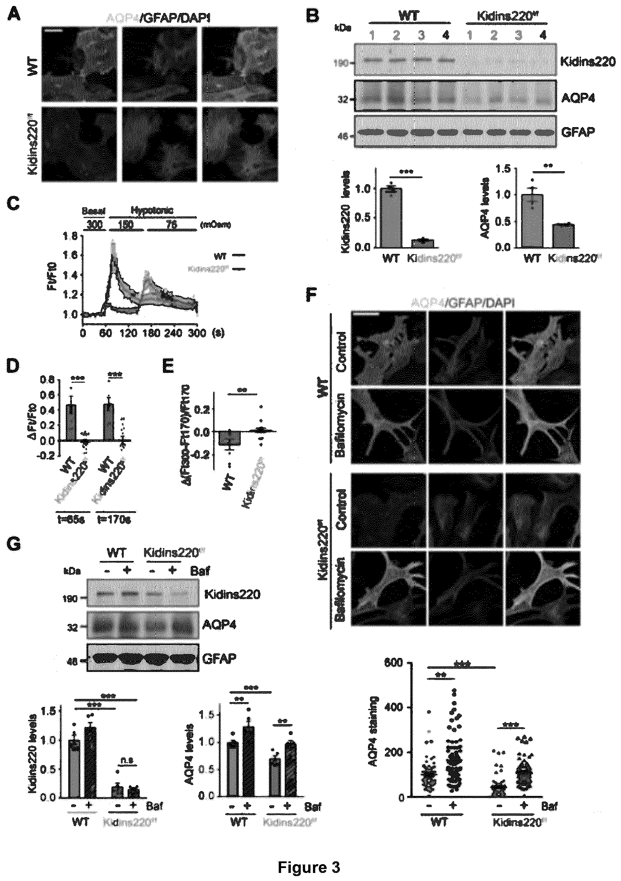

Downregulation of AQP4 in astrocytes from Kidins220 hypomorphic mice. To gain mechanistic insight into the regulation of 3100β and AQP4 expression by Kidins220, we analysed primary cultures of astrocytes because these cells contain high levels of these two molecules and, in contrast to ependymocytes, can be readily cultured, maintained and transfected/transduced in vitro. RTq-PCR showed no significant decreases of Aqp4 or S100β mRNA in cultured astrocytes or in tissue from the PVA obtained from Kidins220^{f/f} animals (not shown). However, we observed a substantial decrease in Kidins220 and AQP4 in cultured astrocytes obtained from Kidins220^{f/f} mice (Fig. 3a, b), suggesting that post-transcriptional mechanisms underlie AQP4 downregulation. Of note, we could not detect 3100β protein.

Astrocytes transiently increase their volume upon hypotonic stress and this process is regulated by AQP4 (Benfenati et al. 2011). To determine whether AQP4 function is impaired in Kidins220^{f/f} astrocytes, we treated astrocytes with a hypotonic stimulus and measured their volume by using the calcein-quenching assay (Benfenati et al. 2011). We found that Kidins220^{f/f} astrocytes did not increase their volume as much as WT cells upon hypotonic stress, and showed a significant delay in bringing their volume back to baseline (Fig. 3c-e). These results strongly suggest that AQP4 function is greatly hampered in Kidins220 deficient astrocytes.

We then investigated the potential post-transcriptional mechanisms involved in AQP4 downregulation. As this water channel can be degraded by the lysosome, we examined the effect of the vATPase inhibitor bafilomycin A1 on AQP4 levels. We found that bafilomycin A1 induced a significant increase in AQP4 levels both in WT and Kidins220^{f/f} astrocytes, as determined by immunofluorescence analysis and immunoblot quantification (Fig. 3f, g). Together, our data strongly suggest that Kidins220 deficiency increases AQP4 lysosomal degradation.

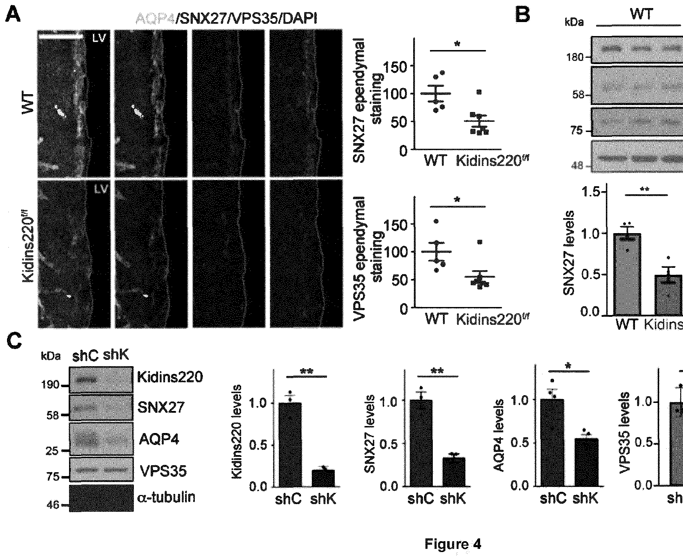

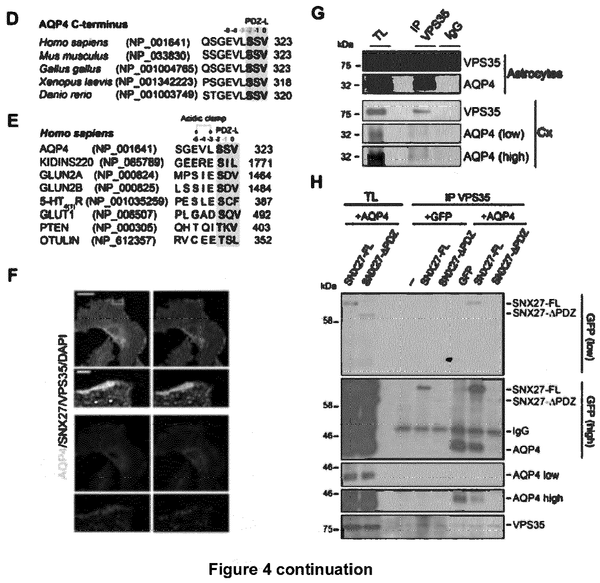

Joint regulation of the SNX27-retromer and its novel cargo AQP4 by Kidins220. Given that Kidins220 endosomal recycling to the cell surface is controlled by the SNX27-retromer complex (Steinberg et al. 2013), and that Snx27 genetic inactivation causes severe postnatal hydrocephalus in mice (Wang et al. 2016), we investigated whether hydrocephalus development in Kidins220 deficient animals could be associated with SNX27-retromer dysfunction. Staining of VPS35, the key SNX-27 retromer component (Cullen et al. 2018), and SNX27 at the ependyma of LVs revealed a prominent decrease of their levels in Kidins220^{f/f} (Fig. 4a). Accordingly, astrocytes from Kidins220^{f/f} mice also showed a severe downregulation of SNX27 and VPS35 (Fig. 4b). We then checked whether Kidins220 could be directly involved in the control of SNX27 and VPS35 by transducing cultured astrocytes from WT mice with lentiviral particles encoding Kidins220 shRNA (shKidins220, referred as shK) or a control shRNA (shC). Strikingly, Kidins220 knockdown strongly reduced SNX27 protein levels without altering those of VPS35 (Fig. 4c). Consistent with results obtained in Kidins220^{f/f} astrocytes, Kidins220 silencing similarly decreased AQP4 content (Fig. 4c).

The loss of SNX27 and VPS35 in Kidins220^{f/f} deficient astrocytes, contrary to that of AQP4, was not rescued by bafilomycin A1 treatment (not shown). In addition, Snx27 and Vps35 mRNA levels did not decrease in PVA tissue or cultured astrocytes from Kidins220^{f/f} mice, while Vps35 transcripts only increased slightly in the glial cells in vitro (not shown), suggesting that post-transcriptional regulation mechanisms distinct from lysosomal degradation mediate Kidins220-dependent SNX27 and VPS35 downregulation.

To address whether AQP4 could be a cargo for the SNX27-retromer, we first analysed the cellular sub-localization of AQP4 and some components of this complex in cultured astrocytes from WT mice. Confocal microscopy imaging showed co-localization of AQP4 not only with SNX27 but also with VPS35 (Fig. 4f). Moreover, AQP4 was present in VPS35-immunocomplexes obtained from murine cerebral cortex and cultured primary astrocytes (Fig. 4g). In addition, we examined AQP4 association to the SNX27-retromer by transfecting HEK293T cells with plasmids encoding AQP4, full-length SNX27 (SNX27-FL) or a mutant lacking its PDZ-domain (SNX27-ΔPDZ), all fused to GFP. Analysis of endogenous VPS35-immunoprecipitates revealed they contained AQP4 (Fig. 4h). Co-expression of SNX27-FL is compatible with the association of AQP4 with VPS35-immunocomplexes but co-expression of SNX27-ΔPDZ disrupted this binding (Fig. 4h). Together, these data indicate that the PDZ domain of SNX27 is necessary for the association of AQP4 with VPS35 and that AQP4 is a novel cargo for the SNX27-retromer.

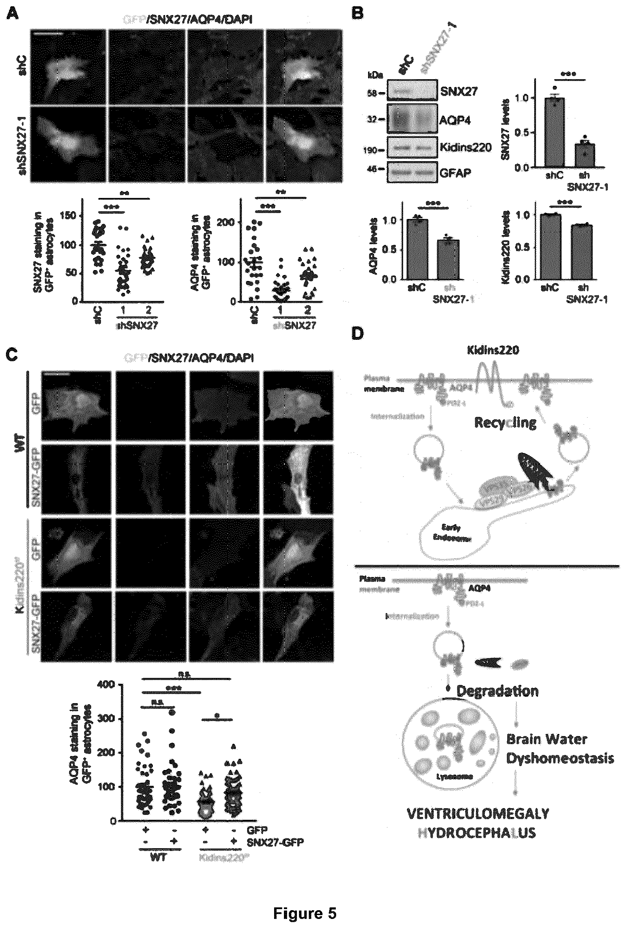

Kidins220-dependent AQP4 downregulation is mediated by SNX27. To investigate whether SNX27 downregulation mediates the decrease of AQP4 levels caused by Kidins220 deficiency, we generated lentiviral particles for SNX27 silencing in WT cultured astrocytes. Transduction of astrocytes with shSNX27 lentivirus sharply decreased AQP4 content, as assessed by immunofluorescence and quantitative immunoblot analysis (Fig. 5a, b). Note that SNX27 knockdown also diminished significantly (P<0.001) Kidins220 protein levels in astrocytes (Fig. 5b), as previously observed in HeLa cells (Steinberg et al. 2013). Conversely, forced SNX27 expression in Kidins220^{f/f} astrocytes substantially increased AQP4 expression (Fig. 5c). Together our data support the notion that Kidins220 deficiency causes SNX27 and VPS35 loss that in turn mediates AQP4 lysosomal degradation and water dyshomeostasis (see scheme in Fig. 5d).

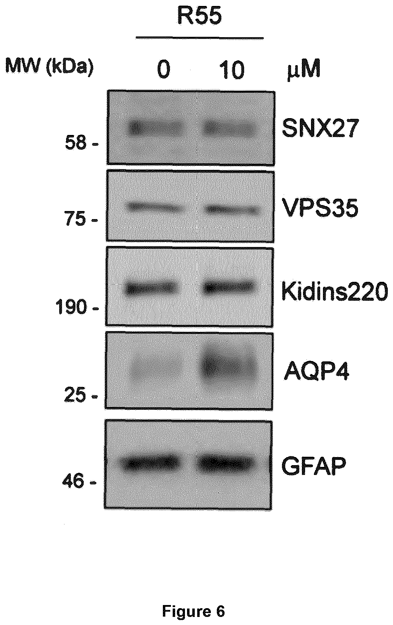

The retromer pharmacological stabilizer R55 increases AQP4 levels in astrocytes. Pharmacological chaperones that stabilize the core retromer complex, interfere its degradation and enhance retromer-mediated trafficking of cargos have been discovered (Mecozzi et al. 2014). Thus, we next explored the effect of one of these drugs, named R55, in the control of the specific and selective SNX27-retromer levels and those of its novel cargo AQP4 in astrocytes. Immunoblot analysis showed that a treatment of 48 h with R55 increased AQP4 content, showing also a mild effect on elevating Kidin220 and VPS35 levels (Fig. 6). These data suggest that stabilization of the retromer can be a good strategy for ventriculomegaly treatment by preventing degradation of the SNX27-retromer and its cargos.

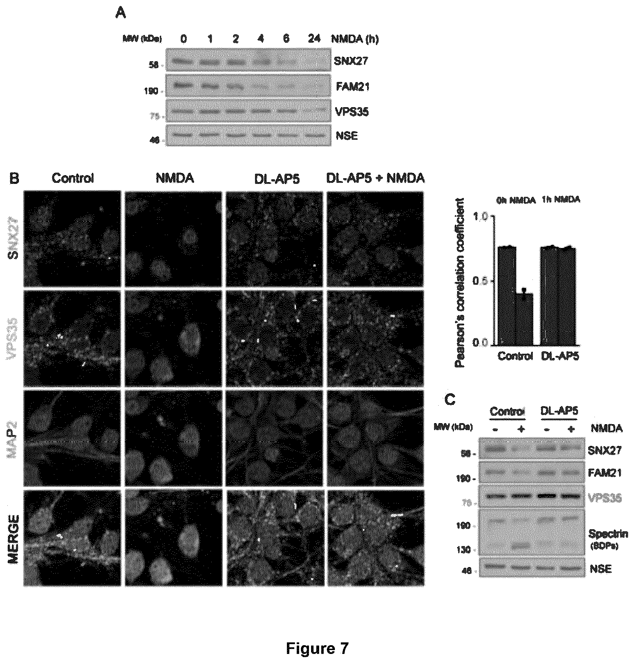

SNX27-retromer dysassembly and degradation in excitotoxic neuronal death. Our data show that Kidins220 deficiency produces SNX27-retromer decreases and targets AQP4 to degradation in astrocytes. We have demonstrated previously that NMDAR-mediated excitotoxicity induces Kidins220 downregulation in neurons (Lopez-Menendez et al. 2009). Therefore, we then questioned whether excitotoxicity could exert similar effects on SNX27-retromer dystabilization and degradation of its neuronal cargos such as Kidins220 and the NMDAR subunits (Cullen et al. 2018). For that purpose, we treated cultures of primary cortical neurons with excitotoxic concentrations of NMDA during different periods of time. We found that excitotoxicity triggered the degradation of SNX27 and that of the SNX27-retromer associated protein FAM21 (Lee et al. 2016) (Fig. 7a). VPS35 levels also decreased at later time points of NMDA treatment (Fig. 7a). Strikingly, excitotoxicity also provoked the early dissociation of SNX27 and VPS35 from intracellular vesicles that could be observed after 1 h of NMDA addition (Fig. 7b). This effect was dependent on NMDAR-overstimulation since pretreatment of neurons for 1 h with the non-competitive NMDAR antagonist DL-AP5 preserved SNX27-retromer intracellular organization and the colocalization of SNX27 and VPS35 as assessed by calculating the Pearson's correlation coefficient (Fig. 7b). Of note, DL-AP5 preincubation, that preserves neuronal survival (Lopez-Menendez et al. 2009), also blocked the degradation of SNX27 and FAM21 induced by 6 h of NMDA treatment (Fig. 7c). Because the protease calpain is activated under excitotoxic conditions, reduced cleavage of the calpain substrate spectrin into breakdown products (BDPs) demonstrates the efficiency of DL-AP5 blocking excitotoxic NMDAR overstimulation.

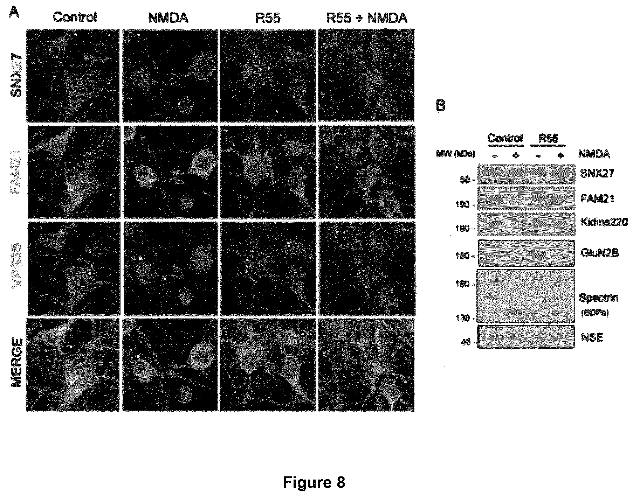

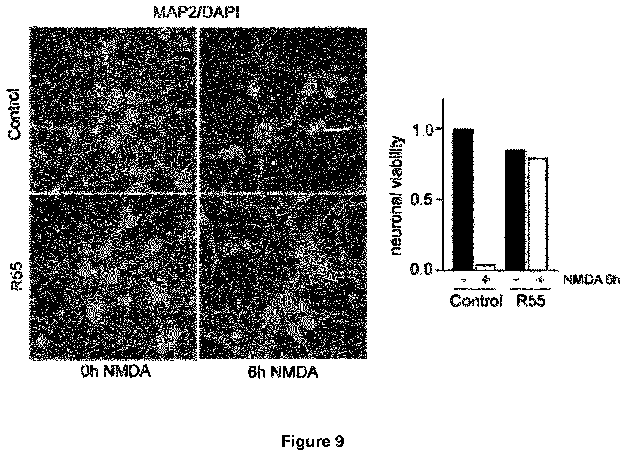

The pharmacological chaperone R55 prevents NMDAR-mediated excitotoxic dissociation and degradation of SNX27-retromer components and cargos, conferring neuroprotection. Next, we tested whether the R55 compound was able to maintain the SNX27-retromer assembly and levels under excitotoxic conditions. As shown in figure 8, pretreatment with R55 preserved SNX27, FAM21 and VPS35 localization in endocytic vesicles in neurons incubated with excitotoxic concentrations of NMDA similarly to that of DL-AP5. Furthermore, R55 effects were very similar to those exerted by DL-AP5 decreasing NMDA-induced degradation of SNX27, FAM21 and the SNX27-retromer cargos Kidins220 and GluN2B subunit of the NMDAR (Fig. 8b). The diminishment in the formation of spectrin BDPs observed in R55 treated neurons suggested this compound could increase neuronal survival. Immunofluorescence staining with the neuronal marker MAP2 and MTT-viability assays demonstrate that indeed R55 treatment conferred a strong neuroprotection to neurons incubated with NMDA for 6h (Fig. 9).

Altogether, these results support that retromer stabilizers are good candidates for therapeutic intervention of diseases that course with Kidins220 dysfunction. and that present ventriculomegaly and/or excitotoxicity.

### Discussion

Here we have uncovered Kidins220-SNX27-retromer as a regulatory pathway for brain AQP4 expression and its involvement in brain ventricular enlargement and hydrocephalus. We have identified a surprising link between SNX27-retromer down-regulation and AQP4 lysosomal degradation induced by Kidins220 deficiency, and discovered that KIDINS220 and AQP4 expression is sharply decreased at the ependymal barrier in iNPH patients. We also show that hypomorphic Kidins220^{f/f} mice are a novel mouse model of hydrocephalus that display ventriculomegaly and a marked AQP4 downregulation in brain astrocytes and in the ependymal cells lining lateral ventricles without apparent ependymocytes structural organization and ciliogenesis impairment.

Ventriculomegaly and excitotoxicity are common signs in several neuropathologies. Importantly, excitotoxicity induces a marked downregulation of Kidins220 in neurons, increasing its degradation and promoting death after NMDAR-overstimulation. Here we discover a decrease in SNX27-retromer components and associated proteins that is linked to degradation of its cargos under NMDAR-induced excitotoxic conditions.

Retromer stabilizers (such as the R55 compound or lentivirus for SNX27 expression) elevate AQP4 levels in astrocytes. On the other hand, in neurons exposed to a highly excitotoxic environment R55 is able to preserve SNX27-retromer organization and levels both of molecules that constitute this retromer (such as SNX27 and FAM21) and also of cargos (such as Kidins220 and the NMDAR subunit GluN2B). Furthermore, R55 confers an elevated neuroprotection from excitotoxicity. Thus, novel strategies designed to potentiate SNX27-retromer activity (including viral vectors, molecules or compounds) could be promising therapeutic approaches to prevent or revert Kidins220 dysfunction.

### REFERENCES

1. Benfenati V, Caprini M, Dovizio M, Mylonakou MN, Ferroni S, Ottersen OP et al. An aquaporin-4/transient receptor potential vanilloid 4 (AQP4/TRPV4) complex is essential for cell-volume control in astrocytes. Proc Natl Acad Sci U S A 2011; 108(6): 2563-2568.
2. Cesca F, Yabe A, Spencer-Dene B, Scholz-Starke J, Medrihan L, Maden CH et al. Kidins220/ARMS mediates the integration of the neurotrophin and VEGF pathways in the vascular and nervous systems. Cell Death Differ 2012; 19(2): 194-208.
3. Cullen PJ, Steinberg F. To degrade or not to degrade: mechanisms and significance of endocytic recycling. Nat Rev Mol Cell Biol2018; 19(11): 679-696.
4. Lee S, Chang J, Blackstone C. FAM21 directs SNX27-retromer cargoes to the plasma membrane by preventing transport to the Golgi apparatus. Nat Commun 2016; 7: 10939.
5. Li JG, Chiu J, Ramanjulu M, Blass BE, Pratico D. A pharmacological chaperone improves memory by reducing Abeta and tau neuropathology in a mouse model with plaques and tangles. Mol Neurodegener2020; 15(1): 1.
6. Lopez-Menendez C, Gascon S, Sobrado M, Vidaurre OG, Higuero AM, Rodriguez-Pena A et al. Kidins220/ARMS downregulation by excitotoxic activation of NMDARs reveals its involvement in neuronal survival and death pathways. J Cell Sci 2009; 122(Pt 19): 3554-3565.
7. Lopez-Menendez C, Simon-Garcia A, Gamir-Morralla A, Pose-Utrilla J, Lujan R, Mochizuki N et al. Excitotoxic targeting of Kidins220 to the Golgi apparatus precedes calpain cleavage of Rap1-activation complexes. Cell Death Dis 2019; 10(7): 535.
8. Mecozzi VJ, Berman DE, Simoes S, Vetanovetz C, Awal MR, Patel VM et al. Pharmacological chaperones stabilize retromer to limit APP processing. Nat Chem Biol 2014; 10(6): 443-449.
9. Muzio L, Sirtori R, Gornati D, Eleuteri S, Fossaghi A, Brancaccio D etal. Retromer stabilization results in neuroprotection in a model of Amyotrophic Lateral Sclerosis. Nat Commun 2020; 11(1): 3848.
10. Papadopoulos MC, Verkman AS. Aquaporin water channels in the nervous system. Nat Rev Neurosci 2013; 14(4): 265-277.
11. Park SH, Eber MR, Tsuzuki S, Booker ME, Sunil AG, Widner DB et al. Adeno-associated virus serotype rh10 is a useful gene transfer vector for sensory nerves that innervate bone in immunodeficient mice. Sci Rep 2017; 7(1): 17428.
12. Steinberg F, Gallon M, Winfield M, Thomas EC, Bell AJ, Heesom KJ et al. A global analysis of SNX27-retromer assembly and cargo specificity reveals a function in glucose and metal ion transport. Nat Cell Biol 2013; 15(5): 461-471.
13. Wang X, Zhou Y, Wang J, Tseng IC, Huang T, Zhao Y et al. SNX27 Deletion Causes Hydrocephalus by Impairing Ependymal Cell Differentiation and Ciliogenesis. J Neurosci 2016; 36(50): 12586-12597.
14. Zhang H, Huang T, Hong Y, Yang W, Zhang X, Luo H et al. The Retromer Complex and Sorting Nexins in Neurodegenerative Diseases. Front Aging Neurosci 2018; 10: 79.

## Claims

1. A retromer complex activator, or a pharmaceutical composition comprising said activator in a therapeutically effective amount, for use in the treatment and/or prevention of neurological or psychiatric disorders **characterized by** a Kinase D-interacting substrate of 220 kDa (Kidins220) dysfunction in a subject.

2. The retromer complex activator or composition for use according to claim 1, wherein the disorder is further **characterized by** ventriculomegaly.

3. The retromer complex activator or composition for use according to claim 1 or 2, wherein the disorder is further **characterized by** excitotoxicity.

4. The retromer complex activator or composition for use according to any one of the preceding claims, wherein the retromer complex activator is an SNX27-retromer (SNX27-R) activator.

5. The retromer complex activator or composition for use according to any one of the preceding claims, wherein the retromer complex activator is thiophen-2-ylmethyl carbamimidothioate hydrochloride (R33) or thiophene-2,5-diylbis(methylene) dicarbamimidothioate dihydrochloride (R55); or variants thereof.

6. The retromer complex activator or composition for use according to any one of claims 1 to 4, wherein the retromer complex activator is selected from the group consisting of: Sorting nexin-27 (SNX27); Vacuolar protein sorting-associated protein 35 (VPS35); Vacuolar protein sorting-associated protein 26 (VPS26); Vacuolar protein sorting-associated protein 29 (VPS29); family with sequence similarity 21 (FAM21); Kinase D-interacting substrate of 220 kDa (KIDINS220); and Aquaporin-4 (AQP4).

7. The retromer complex activator or composition for use according to claim 6, wherein the retromer complex activator is administered to the subject as a nucleic acid.

8. The retromer complex activator or composition for use according to any one of the preceding claims, wherein the subject is a human subject.

9. The retromer complex activator or composition for use according any one of the preceding claims, wherein the neurological or psychiatric disorder is selected from the group consisting of: ischemic stroke (IS); epilepsy; hydrocephalus (HCP); idiopathic Normal Pressure Hydrocephalus (iNPH); schizophrenia; and spastic paraplegia, intellectual disability, nystagmus, and obesity (SINO) syndrome.

10. A method for screening a retromer complex activator for use according to any one of the preceding claims, comprising the following steps:
(i) measuring at least one of the following parameters in a non-human animal:
a. the ventricular volume of the brain ventricles; and/or
b. the levels of expression of at least one gene selected from the group consisting of: *SNX27; VPS35; VPS26*; *VPS29; FAM21*; *KIDINS220;* and *AQP4* in a biological sample from the non-human animal;
(ii) administering a potentially active retromer complex activator for use according to any one of the preceding claims to the non-human animal;
(iii) repeating step (i);
(iv) evaluating the effect of the potentially active retromer complex activator or composition by:
a. comparing the ventricular volumes obtained in (iii)a and (i)a; and/or
b. comparing the expression levels obtained in (iii)b and (i)b, and
(v) selecting the retromer complex activator that stabilizes the retromer complex,
wherein the non-human animal is an animal model with Kidins220 dysfunction; and wherein the retromer complex activator is selected in (v) if the ventricular volumes measured in (iii)a are decreased with respect to the ventricular volumes measured in (i)a, and/or the expression levels of the at least one gene measured in (iii)b are altered with respect to the expression levels measured in (i)b.

11. The method according to claim 10, wherein the non-human animal is a rodent.

12. The method according to claim 10 or 11, wherein the rodent is a Kidins220^{f/f} floxed mouse.

13. An *in vitro* method for the diagnosis of a neurological or psychiatric disorder **characterized by** ventriculomegaly in a human subject, comprising:
(i) measuring the expression levels of at least one protein selected from the group consisting of: *SNX27; VPS35; VPS26*; *VPS29; FAM21; KIDINS220;* and *AQP4* in a biological sample from the human subject, and
(ii) comparing the expression levels measured in (i) with reference expression levels measured in a biological sample from a control human subject,
wherein if the expression levels of the at least one protein listed in (i) are altered with respect to the first reference expression level, then the human subject is suffering from a neurological or psychiatric disorder **characterized by** Kidins220 dysfunction.

14. The *in vitro* method of claim 13, wherein the subject is susceptible to receive a therapy based on the retromer complex activator or composition for use according to any one of claims 1 to 9.

15. The *in vitro* method of any one of claims 13 to 14 wherein the neurological or psychiatric disorder is selected from the group consisting of: IS; epilepsy; HCP; iNPH; schizophrenia; and SINO syndrome.
